(19) Europäisches Patentamt
European Patent Office
Office européen des brevets

(11) EP 1 481 984 A1

(12) # EUROPEAN PATENT APPLICATION

(43) Date of publication:
01.12.2004 Bulletin 2004/49

(21) Application number: 03447130.0

(22) Date of filing: 28.05.2003

(51) Int Cl.[7]: C07K 14/18, A61K 39/29, A61P 31/14, G01N 33/53, G01N 33/576

(84) Designated Contracting States:
AT BE BG CH CY CZ DE DK EE ES FI FR GB GR HU IE IT LI LU MC NL PT RO SE SI SK TR
Designated Extension States:
AL LT LV MK

(71) Applicant: INNOGENETICS N.V.
9052 Gent (BE)

(72) Inventors:
- Depla, Erik
9070 Destelbergen (BE)
- Maertens, Geert
8310 Brugge (BE)
- Bosman, Fons
1745 Opwijk (BE)

Remarks:
Amended claims filed 070804 Claims 11-19 as originally filed are deemed to be abandoned due to non-payment of the claims fee (Rule 31 (2) EPC).

(54) **Modified HEPATITIS C VIRUS (HCV) NS5**

(57) The present invention relates to peptides or polypeptides comprising an HCV NS5 protein or a part thereof or a derivative of said peptide or polypeptide, HCV NS5 protein or part thereof wherein at least one cysteine is reversibly or irreversibly modified. These modified HCV NS5 proteins have advantageous properties both for diagnostic and therapeutic/prophylactic applications.

EP 1 481 984 A1

## Description

### FIELD OF THE INVENTION

The invention relates to the fields of HCV diagnosis, HCV therapeutics and HCV prophylaxis. Specifically, the invention relates to HCV NS5 proteins and their use in the fields mentioned. More specifically the HCV NS5 proteins are modified at their cysteine thiol-groups that are advantageously reversibly or irreversibly protected.

### BACKGROUND TO THE INVENTION

The ca. 9.6 kb single-stranded RNA genome of the HCV virus comprises 5'- and 3'-non-coding regions (NCRs) and, in between these NCRs a single long open reading frame of ca. 9 kb encoding a HCV polyprotein of ca. 3000 amino acids.

HCV polypeptides are produced by translation from the open reading frame followed by proteolytic processing of the resulting ca. 330 kDa polyprotein. Structural proteins are derived from the amino-terminal one-fourth of the polyprotein and include the capsid or Core protein (ca. 21 kDa), the E1 envelope glycoprotein (ca. 31 kDa) and the E2 envelope glycoprotein (ca. 70 kDa), previously called NS1. From the remainder of the HCV polyprotein the non-structural HCV proteins are derived which include NS2 (ca. 23 kDa), NS3 (ca. 70 kDa), NS4A (ca. 8 kDa), NS4B (ca. 27 kDa), NS5A (ca. 58 kDa) and NS5B (ca. 68 kDa) (Grakoui et al. 1993). The E2 protein can occur with or without a C-terminal fusion of the p7 protein (Shimotohno et al. 1995). Recently, an alternative open reading frame in the Core-region was found which is encoding and expressing a ca. 17 kDa protein called F (Frameshift) protein (Xu et al. 2001; Ou & Xu in US Patent Application Publication No. US2002/0076415). In the same region, ORFs for other 14-17 kDa ARFPs (Alternative Reading Frame Proteins), A1 to A4, were discovered and antibodies to at least A1, A2 and A3 were detected in sera of chronically infected patients (Walewski et al. 2001).

HCV is the major cause of non-A, non-B hepatitis worldwide. Acute infection with HCV (20% of all acute hepatitis infections) frequently leads to chronic hepatitis (70% of all chronic hepatitis cases) and end-stage cirrhosis. It is estimated that up to 20% of HCV chronic carriers may develop cirrhosis over a time period of about 20 years and that of those with cirrhosis between 1 to 4%/year is at risk to develop liver carcinoma. (Lauer & Walker 2001, Shiffman 1999). An option to increase the life-span of HCV-caused end-stage liver disease is liver transplantation (30% of all liver transplantations world-wide are due to HCV-infection).

HCV immunoassays, i.e., immunoassays capable of detecting HCV antibodies or antigens (or both), are important in the context of clinical testing as well as in the context of screening of (donated) blood and its derivatives. In a clinical test, body fluid (e.g., serum) or a solid sample of a body (e.g., liver biopsy) is diagnosed for the presence of HCV, is monitored for the course of HCV disease development and/or is monitored for the effect of a treatment in a HCV-infected individual. Large-scale screening of blood and its derivatives for the presence of HCV (as well as of, e.g., HIV and HBV) is required by regulatory authorities. As a result thereof the supply of blood or its derivatives free from pathogenic contaminants can be safeguarded.

HCV immunoassays may be divided in screening assays and confirmation assays. Preferably, the confirmation assays comprise a different set of antigens (in case of anti-HCV-antibody detection) than the set of antigens in the screening assays.

WO91/15771 discloses a combination of a Core antigen with at least one of an envelope, NS3, NS4 or NS5 antigen. The preferred NS3 antigen herein is C33c (see above) whereas the preferred NS5 antigen spans amino acids 2054-2464 of the HCV polyprotein. The latter NS5 antigen is also obtained as a fusion protein with an N-terminal SOD fragment. NS5 production is referred to in Example 5 of WO91/15771 to be similar to the production of the NS3 C33c antigen as described in Example 1 of WO91/15771. From the latter Example it is clear that cysteine-thiol groups are not protected.

NS5 peptide antigens are disclosed in WO92/10514 and WO93/18054.

A HCV vaccine for prophylactic and/or therapeutic purposes may be a DNA-based vaccine, a protein- or peptide-based vaccine, or a combination of a DNA-prime protein-boost vaccination may be applied. Only vaccinations including proteins or peptides are listed below.

DNA-prime protein-boost vaccination studies have been performed in mice for Core (Hu et al. 1999) and E2 (Song et al. 2000).

Studies with protein- or peptide-based HCV vaccines, i.e. subunit HCV vaccines, are very limited and include immunization of mice with fragments of Core (Shirai et al. 1996, Hu et al. 1999), E1 (Lopez-Diaz de Cerio et al. 1999), E2 (Nakano et al. in US Patent Publication No. 2002/0119495; Houghton et al. in US Patent Application Publication No. 2002/0002272), E1/E2 or E1/E2 + Core (Drane et al. in International Patent Publication No. WO01/37869) and NS5 (Shirai et al. 1996, Uno-Furuta et al. 2001).

All of the above exploratory vaccinations were performed on rodents. Only a limited number of prophylactic and therapeutic vaccinations of primates or chimpanzees or therapeutic vaccinations of HCV-infected humans have been performed. E2 DNA-vaccinations of mice, macaques and chimpanzees were described in two studies of Forns et al. (1999, 2000). Rhesus macaques were injected with Core-expressing vaccinia virus, Core adjuvanted with LTK63 or Core adjuvanted with ISCOM in a study by Drane et al. (in International Patent Publication No. WO01/37869). Prophylactic vaccination of chimpanzees with an E1/E2 or Core/E1/E2 complex has been described in Choo et al. (1994), Houghton et al. (1995). Prophylactic and therapeutic vaccination of chimpanzees with an E1 protein has been described in WO99/67285 and WO02/055548. Interestingly, the immune responses observed in chimpanzees were also observed in HCV-infected humans and in healthy volunteers.

From the above, it will be clear that not much effort has been invested to date in optimizing the diagnostic properties of HCV NS5 proteins. It will also be clear that the immunogenic properties of HCV NS5 have been scarcely explored. The present invention discloses HCV NS5 proteins modified in two different ways. These modified HCV NS5 proteins both display increased sensitivity, compared to the art, in diagnostic assays and, in addition thereto, are for the first time shown to be capable of inducing an encouraging immunogenic response, hence opening the way to their incorporation in HCV vaccine compositions.

## SUMMARY OF THE INVENTION

In a first aspect the current invention relates to an isolated HCV NS5 protein or a part thereof wherein at least one cysteine thiol groups is chemically or enzymatically modified. Further part of the invention are derivatives of an HCV NS5 protein or a part thereof wherein at least one of cysteine thiol groups is chemically or enzymatically modified. In a specific embodiment thereto, at least one cysteine thiol group in said isolated protein or part thereof or derivative of any thereof is chemically or enzymatically modified in a reversible or irreversible fashion. In a further specific embodiment, said modification in a reversible fashion is a chemical or enzymatic modification by reversible sulfonation or said modification in an irreversible fashion is a chemical modification by irreversible alkylation.

Another aspect of the current invention relates to a composition comprising an isolated protein or part thereof or derivative of any thereof according to the invention and at least one of a pharmaceutically acceptable carrier, adjuvant or vehicle. In specific embodiments thereto, said composition is a HCV immunogenic composition, a prophylactic HCV vaccine composition or a therapeutic HCV vaccine composition. Any of said compositions may further comprise a DNA vaccine vector, in particular a HCV DNA vaccine vector.

Another aspect of the current invention relates to the use of an isolated protein or part thereof or derivative of any thereof according to the invention for the manufacture of a HCV immunogenic composition, a prophylactic HCV vaccine composition or a therapeutic HCV vaccine composition.

Further aspects of the current invention comprise the HCV immunogenic composition, an HCV vaccine composition, a prophylactic HCV vaccine composition and/or a therapeutic HCV vaccine composition according to the invention for; or alternatively comprises the use of any of said compositions for:

- inducing in a mammal a humoral response to the HCV peptides comprised in any of said compositions; and/or
- inducing in a mammal a cellular response to the HCV peptides comprised in any of said compositions, wherein said cellular response may be a $CD4^+$ T-cell proliferation response and/or a $CD8^+$ cytotoxic T-cell response and/or the increased production of cytokines; and/or
- prophylactic protection of a mammal against chronic HCV infection, wherein said HCV infection may be a homologous or a heterologous HCV infection; and/or
- therapeutically treating a chronically HCV-infected mammal, wherein said HCV may be a homologous or a heterologous HCV; and/or
- reducing liver disease in a HCV-infected mammal; and/or
- reducing liver disease in a chronic HCV-infected mammal by at least 2 points according to the overall Ishak score; and/or
- reducing serum liver enzyme activity levels in a HCV-infected mammal, wherein said liver enzyme may be, e.g., alanine aminotransferase (ALT) or gamma-glutamylpeptidase; and/or
- reducing HCV RNA levels in a HCV-infected mammal; and/or
- reducing liver fibrosis progression in a HCV-infected mammal; and/or
- reducing liver fibrosis in a HCV-infected mammal; and/or
- reducing HCV antigen levels in or presented on liver cells, wherein said HCV antigens include E2 or Core antigens.

Another aspect of the current invention relates to the use of an isolated protein or a part thereof or a derivative of any thereof according to the invention in immunoassays, to the incorporation of an isolated protein or part thereof or derivative of any thereof according to the invention in immunoassay kits or diagnostic kits, and to the use of an isolated protein or part thereof or derivative of any thereof according to the invention for the manufacture of an immunoassay kit or diagnostic kit. Immunoassays comprise immunological methods for determining the presence of antibodies to HCV in a biological sample or of antigens of HCV in a biological sample or of HCV virus in a biological sample, or for diagnosing HCV infection. Diagnostic kits or immunoassay kits comprise kits for determining the presence of antibodies to HCV in a biological sample or of antigens of HCV in a biological sample or of HCV virus in a biological sample, or for diagnosing HCV infection.

A first general embodiment in relation to immunoassays comprises a method for determining the presence of antibodies to HCV, in particular to HCV NS5, in a biological sample comprising the step of detecting said antibodies to an isolated protein or part thereof or derivative of any thereof according to the invention.

A second general embodiment in relation to immunoassays comprises a method for determining the presence of HCV NS5 antigens in a biological sample comprising the step of detecting said HCV NS5 antigens with an antibody to said HCV NS5 antigens in the presence of an isolated protein or part thereof or derivative of any thereof according to the invention as competitor of binding of said HCV NS5 antigens to said antibody.

In particular said immunoassays relying on an isolated protein or part thereof or derivative of any thereof according to the invention wherein at least one cysteine is reversibly modified are performed in the absence of a reducing agent.

The invention further relates to a method for producing an HCV NS5 protein or part thereof or derivative of any thereof according to the invention wherein said method comprises the steps of:

(i) obtaining an HCV NS5 protein or part thereof by means of recombinant expression or chemical synthesis;
(ii) reversibly or irreversibly modifying at least one cysteine thiol group in the HCV NS5 protein or part thereof obtained in (i);
(iii) purifying the HCV NS5 protein or part thereof of (ii).

## FIGURE LEGENDS

**Figure 1.** Schematic map of the vector pIGFH113NS5a.

**Figure 2.** Log EC50 values of antibody titers induced in mice upon immunization with sulfonated (A) or alkylated (B) HCV NS5A. The ELISA was performed with either alkylated (IAA) or desulfonated (SO3) HCV NS5A as coated reagent as indicated in the X-axis. The horizontal lines represent mean values. The underlying experiment is outlined in Example 5 herein.

**Figure 3.** Stimulation Index (SI) values, reflecting the cellular immune response induced in mice upon immunization with sulfonated (A) or alkylated (B) HCV NS5A. The in vitro restimulation was performed with either alkylated (IAA) or sulfonated (SO3) HCV NS5A as indicated in the X-axis. The horizontal lines represent mean values. The underlying experiment is outlined in Example 5 herein.

## DETAILED DESCRIPTION OF THE INVENTION

The present invention relates to the diagnostic and immunogenic properties of HCV NS5 proteins wherein at least one cysteine has been reversibly or irreversibly modified. Cysteines in HCV NS5 have more specifically been sulfonated or alkylated yielding NS5-SO3 or NS5-IAA, respectively.

In WO91/15771, the NS5 antigen (spanning amino acids 2054-2464 of the HCV polyprotein; comprised in a fusion protein with N-terminal SOD fragment) yielded 57% (chronic HCV carrier serum samples only) or 51% (all HCV carrier serum samples) HCV-positive scores under conditions where no reducing agent was present in the assay (see Table 1 in WO91/15771). From the healthy donor serum samples 18.5% tested HCV-positive, i.e. false positive, with the NS5 antigen under the same conditions (see Table 2 in WO91/15771). The diagnostic performance of an HCV NS5-SO3 and an HCV NS5-IAA antigen according to the present invention has been explored as outlined in Example 4 and Table 2 herein. From Tables 1 (ELISA with NS3; see Example 3) and 2 (ELISA with NS5) of the current invention it will be clear that a number of serum samples occur in both Tables. In an ELISA with either one of the NS5-SO3 protein or NS5-IAA protein of the current invention as much as 92% of the serum samples also tested in an NS3 ELISA (and therein found HCV-positivie; compare Tables 1 and 2) were found to be HCV-positive. The exceptions are HCV carrier serum Nos. 17794 and 17808. Of all serum samples tested and listed in Table 2, 86% react positive with NS5-SO3 or NS5-IAA (when considering serum No 17758 as negative). Furthermore, only one, i.e. 4%, of the healthy donor serum samples tested potentially HCV-positive with either of said NS5 proteins (i.e. donor serum No. F526; see Table 2). These results are thus clearly superior to the results with the NS5 antigen as described in WO91/15771.

In contrast to what is known for HCV NS3, a reducing agent does not need to be present for obtaining a sensitive NS5-antigen based diagnostic assay (apparent from comparison of Examples 3 and 4, and Tables 1 and 2 herein). Thus potential toxic effects of reducing agents are eliminated as well as the unpleasant smell of such agents. For example, 2-mercaptoethanol is toxic by inhalation, ingestion and through skin contact, is a severe eye irritant and is readily absorbed through the skin (info from a material safety data sheet).

To date the immunogenic properties of HCV NS5 proteins have not been extensively explored. This may well be due to the considerable problems related to obtaining stable HCV NS5 protein in sufficient quantities. The modifications applied to the HCV NS5 protein as subject of the present invention are overcoming this problem. The modified HCV NS5 proteins of this invention are moreover, and despite of or due to their modification, surprisingly good immunogens (see Example 5 and Figures 2 and 3hererin), a prerequisite for their use in therapeutic and/or prophylactic applications.

In a first aspect the current invention thus relates to an isolated HCV NS5 protein or part thereof or derivative of any thereof wherein at least one cysteine thiol groups is chemically or enzymatically modified. In a specific embodiment thereto, at least one cysteine thiol group in said isolated protein or part thereof or derivative of any thereof is chemically or enzymatically modified in a reversible or irreversible fashion. In a further specific embodiment, said modification in a reversible fashion is a chemical or enzymatic modification by reversible sulfonation or said modification in an irreversible fashion is a chemical modification by irreversible alkylation.

The terms peptide, polypeptide and protein are used interchangeably herein.

A derivative of a protein of the invention, e.g. an HCV NS5 protein or a part thereof, is meant to include proteins comprising derivatized amino acids (e.g., conjugated with biotin or digoxigenin), non-natural amino acids, HCV NS5 proteins comprising insertions, deletions or substitutions (relative to a naturally occurring HCV NS5 sequence) of one or more amino acids, as well as fusion proteins. A derivatized amino acid includes a derivatized cysteine wherein the derivatization is a modification of the thiol group and/or another modification. Fusion proteins may be formed between two distinct HCV peptides or between an HCV NS5 peptide and another peptide or protein such as a B-cell epitope, a T-cell epitope, a CTL epitope or a cytokine. Other peptide or protein fusion partners include bovine serum album, keyhole limpet hemocyanin, soybean or horseradish peroxidase, beta-galactosidase, luciferase, alkaline phosphatase, glutathione S-transferase or dihydrofolate reductase or heterologous epitopes such as (histidine)$_6$-tag, protein A, maltose-binding protein, Tag•100 epitope, c-myc epitope, FLAG® -epitope, lacZ, CMP (calmodulin-binding peptide), HA epitope, protein C epitope or VSV epitope. Other proteins include histones, single-strand binding protein (ssB) and native and engineered fluorescent proteins such as green-, red-, blue-, yellow-, cyan-fluorescent proteins.

The HCV NS5 protein corresponds to the HCV polyprotein region spanning amino acids 1973-3011. The HCV NS5 protein is usually further divided in two regions, the NS5A and NS5B regions, spanning amino acids 1973-2420 and 2421-3011 of the HCV polyprotein, respectively. It is to be understood that these endpoints are approximations. The mentioned endpoints are not absolute as they may vary, e.g., due to insertions/deletions in an upstream part of the HCV polyprotein or in the HCV NS5 region itself. Such insertions/deletions are known to be present as is apparent when HCV polyprotein sequences of different genotypes are compared. With a part of an HCV NS5 protein is meant any part that comprises at least one cysteine residue, alternatively said part comprises 2, 3, 4, 5, 6, 7, 8, 9, 10, 11, 12, 13, 14, 15, 16, 17, 18, 19, 20 or up to all cysteine (naturally occurring and/or introduced, see further) residues of an HCV NS5 protein, the current upper limit of genotype-, subtype- or isolate-dependent number of cysteine residues in a naturally occurring HCV NS5A protein being 18. Said cysteine residue may be either a naturally occurring cysteine residue or a non-naturally occurring cysteine residue introduced, e.g., by genetic engineering or during synthetic protein manufacturing. Preferably, said part of HCV NS5 comprises at least one HCV NS5 epitope (B-cell epitope or T-cell epitope).

In a specific embodiment, the peptide or polypeptide of the invention or derivative thereof is comprising the HCV polyprotein region spanning amino acids 1973-2420, i.e. an HCV NS5A peptide or polypeptide. In a further specific embodiment, the peptide or polypeptide of the invention or a derivative thereof is comprising the HCV polyprotein region spanning amino acids 1973-2420 defined by SEQ ID NO:4. An exemplary peptide or polypeptide of the invention is defined by SEQ ID NO:3. For recombinant expression purposes, an amino-terminal methionine may be included in the peptide or polypeptide of the invention.

A further aspect of the invention relates to a method for producing an HCV NS5 protein or part thereof or derivative of any thereof according to the invention wherein said method comprises the steps of:

(i) obtaining an HCV NS5 protein or part thereof by means of recombinant expression or chemical synthesis;
(ii) reversibly or irreversibly modifying at least one cysteine thiol group in the HCV NS5 protein or part thereof obtained in (i);
(iii) purifying the HCV NS5 protein or part thereof of (ii).

Any of the proteins, parts thereof or derivatives of any thereof according to the present invention may be of synthetic origin, i.e. synthesized by applying organic chemistry, or of recombinant origin. HCV peptides may be produced by expression in, e.g., mammalian or insect cells infected with recombinant viruses, yeast cells or bacterial cells.

More particularly, said mammalian cells include HeLa cells, Vero cells, RK13 cells, MRC-5 cells, Chinese hamster ovary (CHO) cells, Baby hamster kidney (BHK) cells and PK15 cells. More particularly, said insect cells include cells of *Spodoptera frugiperda*, such as Sf9 cells. More particularly, said recombinant viruses include recombinant vaccinia viruses, recombinant adenoviruses, recombinant baculoviruses, recombinant canary pox viruses, recombinant Semliki Forest viruses, recombinant alphaviruses, recombinant Ankara Modified viruses and recombinant avipox viruses. More particularly, said yeast cells include cells of *Saccharomyces,* such as *Saccharomyces cerevisiae, Saccharomyces kluyveri,* or *Saccharomyces uvarum, Schizosaccharomyces,* such as *Schizosaccharomyces pombe, Kluyveromyces,* such as *Kluyveromyces lactis, Yarrowia,* such as *Yarrowia lipolytica, Hansenula,* such as *Hansenula polymorpha, Pichia,* such as *Pichia pastoris, Aspergillus species, Neurospora,* such as *Neurospora crassa,* or *Schwanniomyces,* such as *Schwanniomyces occidentalis,* or mutant cells derived from any thereof. More specifically, the HCV peptide or part thereof according to the invention is the product of expression in a *Hansenula* cell. More particularly, said bacterial cells include cells of *Escherichia coli* or *Streptomyces* species.

An epitope is referring to a structure capable of binding to and/or activating a cell involved in eliciting an immune response to said structure. Epitopes thus include epitopes of B-cells, T-cells, T-helper cells and CTLs. Epitopes include conformational epitopes and linear epitopes. Peptide-or protein-epitopes comprise peptides or parts of peptides or proteins capable of binding to, e.g., T-cell receptors, B-cell receptors, antibodies or MHC molecules. The size of linear peptide- or protein-epitopes can be limited to a few, e.g. 4, 5, 6, 7, 8, 9, 10, 11, 12, 13, 14 or 15 amino acids. An epitope is antigenic but not always immunogenic.

A T-cell stimulating epitope refers to an epitope capable of stimulating T-cells, T-helper cells or CTL-cells. A T-helper cell stimulating epitope may be selected by monitoring the lymphoproliferative response, also referred to as $CD4^+$ T-cell proliferation response, towards (potential antigenic) polypeptides containing in their amino acid sequence a (putative) T-cell stimulating epitope. Said lymphoproliferative response may be measured by either a T-helper assay comprising *in vitro* stimulation of peripheral blood mononuclear cells (PBMCs) from patient sera with varying concentrations of peptides to be tested for T-cell stimulating activity and counting the amount of radiolabelled thymidine taken up by the PBMCs. Proliferation is considered positive when the stimulation index (mean cpm of antigen-stimulated cultures/mean cpm of controle cultures) is more than 1, preferably more than 2, most preferably more than 3. A CTL-stimulating epitope may be selected by means of a cytotoxic T-lymphocyte or cytotoxic T-cell (CTL) assay measuring the lytic activity of cytotoxic cells, also referred to as $CD8^+$ CTL response, using $^{51}Cr$ release. Cell-mediated responses may also be assessed by measuring cytokine production, e.g., by an ELISpot assay (see for instance Fujihashi et al. 1993). Characteristic for a Th1-like response is the production/secretion of, e.g., IL-2 and/or IFN-$\gamma$. Characteristic for a Th2-like response is the production/secretion of, e.g., IL-4.

In the protein, part thereof or derivative of any thereof comprising at least one cysteine residue, the cysteine thiol-group(s) can be irreversibly protected by chemical means. "Irreversible protection" or "irreversible blocking" by chemical means refers to alkylation, preferably alkylation of the HCV NS5 proteins by means of alkylating agents, such as, for example, active halogens, ethylenimine or N-(iodoethyl)trifluoro-acetamide. In this respect, it is to be understood that alkylation of cysteine thiol-groups refers to the replacement of the thiol-hydrogen by $(CH_2)_nR$, in which n is 0, 1, 2, 3 or 4 and R= H, COOH, $NH_2$, $CONH_2$, phenyl, or any derivative thereof. Alkylation can be performed by any method known in the art, such as, for example, active halogens $X(CH_2)_nR$ in which X is a halogen such as I, Br, Cl or F. Examples of active halogens are methyliodide, iodoacetic acid, iodoacetamide, and 2-bromoethylamine. Other methods of alkylation include the use of NEM (N-ethylmaleimide) or Biotin-NEM, a mixture thereof, or ethylenimine or N-(iodoethyl) trifluoroacetamide both resulting in substitution of -H by $-CH_2-CH_2-NH_2$ (Hermanson, G. T. 1996). The term "alkylating agents" as used herein refers to compounds which are able to perform alkylation as described herein.

It is further understood that the cysteine thiol-groups of the HCV proteins, i.e. HCV NS5 proteins, or the parts thereof or the derivatives of any thereof of the present invention can be reversibly protected. The purpose of reversible protection is to stabilize the HCV protein or part thereof or derivative of any thereof. Especially, after reversible protection the sulfur-containing functional group (e.g. thiols and disulfides) is retained in a non-reactive condition. The sulfur-containing functional group is thus unable to react with other compounds, e.g. have lost their tendency of forming or exchanging disulfide bonds, such as, for example

$$R_1\text{-SH} + R_2\text{-SH} \text{ ---X---> } R_1\text{-S-S-}R_2 \ ;$$

$$R_1\text{-S-S-}R_2 + R_3\text{-SH} \text{ ---X---> } R_1\text{-S-S-}R_3 + R_2\text{-SH} \ ;$$

$$R_1\text{-S-S-}R_2 + R_3\text{-S-S-}R_4 \text{ ---X---> } R_1\text{-S-S-}R_3 + R_2\text{-S-S-}R_4.$$

The described reactions between thiols and/or disulfide residues are not limited to intermolecular processes, but may also occur intramolecularly.

The term "reversible protection" or "reversible blocking" as used herein contemplates covalently binding of modification agents to the cysteine thiol-groups, as well as manipulating the environment of the HCV protein such, that the redox state of the cysteine thiol-groups remains unaffected throughout subsequent steps of the purification procedure (shielding). Reversible protection of the cysteine thiol-groups can be carried out chemically or enzymatically.
The term "reversible protection by enzymatical means" as used herein contemplates reversible protection mediated by enzymes, such as for example acyl-transferases, e.g. acyl-transferases that are involved in catalysing thio-esterification, such as palmitoyl acyltransferase (see below).
The term "reversible protection by chemical means" as used herein contemplates reversible protection:

1. by modification agents that reversibly modify cysteinyls such as for example by sulfonation and thio-esterification; Sulfonation is a reaction where thiol or cysteines involved in disulfide bridges are modified to *S*-sulfonate: RSH $\rightarrow$ $RS\text{-}SO_3^-$ (Darbre, A. 1986) or RS-SR$\rightarrow$ 2 $RS\text{-}SO_3^-$ (sulfitolysis; (Kumar, N. et al. 1986)). Reagents for sulfonation are e.g. $Na_2SO_3$, or sodium tetrathionate. The latter reagents for sulfonation are used in a concentration of 10-200 mM, and more preferentially in a concentration of 50-200 mM. Optionally sulfonation can be performed in the presence of a catalysator such as, for example $Cu^{2+}$ (100 µM-1 mM) or cysteine (1-10 mM).
The reaction can be performed under protein denaturing as well as native conditions (Kumar, N. et al. 1985, Kumar, N. et al. 1986).
Thioester bond formation, or thio-esterification is characterised by:

$$RSH + R'COX \rightarrow RS\text{-}COR'$$

in which X is preferentially a halogenide in the compound R'CO-X.
2. by modification agents that reversibly modify the cysteinyls of the present invention such as, for example, by heavy metals, in particular $Zn^{2+}$, $Cd^{2+}$, mono-, dithio- and disulfide-compounds (e.g. aryl- and alkylmethanethiosulfonate, dithiopyridine, dithiomorpholine, dihydrolipoamide, Ellmann reagent, aldrothiol™ (Aldrich) (Rein, A. et al. 1996), dithiocarbamates), or thiolation agents (e.g. gluthathion, N-Acetyl cysteine, cysteineamine). Dithiocarbamate comprise a broad class of molecules possessing an $R_1R_2NC(S)SR_3$ functional group, which gives them the ability to react with sulfydryl groups. Thiol containing compounds are preferentially used in a concentration of 0.1-50 mM, more preferentially in a concentration of 1-50 mM, and even more preferentially in a concentration of 10-50 mM;
3. by the presence of modification agents that preserve the thiol status (stabilise), in particular antioxidantia, such as for example DTT, dihydroascorbate, vitamins and derivates, mannitol, amino acids, peptides and derivates (e.g. histidine, ergothioneine, carnosine, methionine), gallates, hydroxyanisole, hydoxytoluene, hydroquinon, hydroxymethylphenol and their derivates in concentration range of 10 µM-10 mM, more preferentially in a concentration of 1-10 mM;
4. by thiol stabilising conditions such as, for example, (i) cofactors as metal ions ($Zn^{2+}$, $Mg^{2+}$), ATP, (ii) pH control (e.g. for proteins in most cases pH ~5 or pH is preferentially thiol $pK_a$ -2; e.g. for peptides purified by Reversed Phase Chromatography at pH ~2).

Combinations of reversible protection as described in (1), (2), (3) and (4) may result in similarly pure and refolded HCV proteins. In effect, combination compounds can be used, such as, for example Z103 (Zn carnosine), preferentially in a concentration of 1-10 mM. It should be clear that reversible protection also refers to, besides the modification groups or shielding described above, any cysteinyl protection method which may be reversed enzymatically or chemically, without disrupting the peptide backbone. In this respect, the present invention specifically refers to peptides prepared by classical chemical synthesis (see above), in which, for example, thioester bounds are cleaved by thioesterase, basic buffer conditions (Beekman, N. J. et al. 1997) or by hydroxylamine treatment (Vingerhoeds, M. H. et al. 1996).
Reversible protection may also be used to increase the solubilisation and extraction of peptides (Pomroy, N. C. and Deber, C. M. 1998).
The reversible protection and thiol stabilizing compounds may be presented under a monomeric, polymeric or liposomic form.
The removal of the reversibly protection state of the cysteine residues can chemically or enzymatically accomplished by e.g.:

- a reductant, in particular DTT, DTE, 2-mercaptoethanol, dithionite, $SnCl_2$, sodium borohydride, hydroxylamine, TCEP, in particular in a concentration of 1-200 mM, more preferentially in a concentration of 50-200 mM;
- removal of the thiol stabilising conditions or agents by e.g. pH increase;

- enzymes, in particular thioesterases, glutaredoxine, thioredoxine, in particular in a concentration of 0.01-5 µM, even more particular in a concentration range of 0.1-5 µM.;
- combinations of the above described chemical and/or enzymatical conditions.

The removal of the reversibly protection state of the cysteine residues can be carried out *in vitro* or *in vivo,* e. g. in a cell or in an individual.

It will be appreciated that in the purification procedure, the cysteine residues may or may not be irreversibly blocked, or replaced by any reversible modification agent, as listed above. Reversibly blocked cysteines in a protein may be converted to irreversibly blocked cysteines.

A reductant according to the present invention is any agent which achieves reduction of the sulfur in cysteine residues, e.g. "S-S" disulfide bridges, desulfonation of the cysteine residue ($RS\text{-}SO_3^- \rightarrow RSH$). An antioxidant is any reagent which preserves the thiol status or minimises "S-S" formation and/or exchanges. Reduction of the "S-S" disulfide bridges is a chemical reaction whereby the disulfides are reduced to thiol (-SH). "S-S" Reduction can be obtained by (1) enzymatic cascade pathways or by (2) reducing compounds. Enzymes like thioredoxin, glutaredoxin are known to be involved in the in vivo reduction of disulfides and have also been shown to be effective in reducing "S-S" bridges in vitro. Disulfide bonds are rapidly cleaved by reduced thioredoxin at pH 7.0, with an apparent second order rate that is around $10^4$ times larger than the corresponding rate constant for the reaction with DTT. The reduction kinetic can be dramatically increased by preincubation the protein solution with 1 mM DTT or dihydrolipoamide (Holmgren, A. 1979). Thiol compounds able to reduce protein disulfide bridges are for instance Dithiothreitol (DTT), Dithioerythritol (DTE), β-mercaptoethanol, thiocarbamates, bis(2-mercaptoethyl) sulfone and N,N'-bis(mercaptoacetyl)hydrazine, and sodium-dithionite. Reducing agents without thiol groups like ascorbate or stannous chloride ($SnCl_2$), which have been shown to be very useful in the reduction of disulfide bridges in monoclonal antibodies (Thakur, M. L. et al. 1991), may also be used for the reduction of HCV proteins. In addition, changes in pH values may influence the redox status of HCV proteins. Sodium borohydride treatment has been shown to be effective for the reduction of disulfide bridges in peptides (Gailit, J. 1993). Tris (2-carboxyethyl)phosphine (TCEP) is able to reduce disulfides at low pH (Bums, J. et al. 1991). Selenol catalyses the reduction of disulfide to thiols when DTT or sodium borohydride is used as reductant. Selenocysteamine, a commercially available diselenide, was used as precursor of the catalyst (Singh, R. and Kats, L. 1995).

Another aspect of the current invention relates to a composition comprising an isolated protein or part thereof or derivative of any thereof according to the invention (see first aspect of the invention) and at least one of a pharmaceutically acceptable carrier, adjuvant or vehicle. In specific embodiments thereto, said composition is a HCV immunogenic composition, a prophylactic HCV vaccine composition or a therapeutic HCV vaccine composition. In particular a HCV immunogenic composition, a prophylactic HCV vaccine composition or a therapeutic HCV vaccine composition comprises an effective amount of an isolated protein or part thereof or derivative of any thereof according to the invention. Any of the listed compositions may further comprise a DNA vaccine vector, e.g., a HCV DNA vaccine vector.

The term "immunogenic" refers to the ability of a protein or a substance to produce at least one element of an immune response. The immune response is the total response of the body of an animal to the introduction of an antigen and comprises multiple elements including antibody formation (humoral response or humoral immunity), cellular immunity, hypersensitivity, or immunological tolerance. Cellular immunity refers to cellular responses elicited by an antigen and include a T-helper cell- and/or CTL-response and/or stimulated cytokine production. The term "antigen" refers to the ability of a peptide, protein or other substance to be antigenic or immunogenic. An antigen is understood to comprise at least one epitope.

"Antigenic" refers to the capability of a protein or substance to be recognized by an elicited humoral and/or cellular immune response. Typically, the antigenic quality of a protein or substance is determined by in vitro assays. For humoral responses, a protein or substance can be referred to as antigenic in case the protein or substance is recognized by elicited antibodies in e.g. an ELISA, western-blot, RIA, immunoprecipitation assay or any similar assay in which the protein or substance is allowed to be recognized by an elicited antibody and in which such a recognition can be measured by, e.g., a colorometric, fluorometric or radioactive detection, or formation of a precipitate. For cellular response, a protein or substance can be referred to as antigenic in case the protein or substance is recognized by an elicited T-cell response in e.g. an T-cell proliferation assay, a $^{51}Cr$-release assay, a cytokine secretion assay or alike in which the protein or substance is incubated in the presence of T-cells drawn from an individual in which immune response have been elicited and in which a recognition by the T-cell is measured by, e.g., a proliferative response, a cell lysis response, a cytokine secretion. An antigenic protein or substance may be immunogenic in se but may also require additional structures to be rendered immunogenic.

An "immunogenic composition" is a composition comprising an antigen capable of eliciting at least one element of the immune response against the antigen comprised in said composition when said composition is introduced into the body of an animal capable of raising an immune response. An immunogenic composition may comprise more than one antigen, i.e., a plurality of antigens, e.g. 2, 3, 4, 5, 6, 7, 8, 9, 10 or more, e.g., up to 15, 20, 25, 30, 40 or 50 or more distinct antigens. In particular, the immunogenic composition of the invention is an HCV immunogenic composition wherein the antigen or plurality of antigens are peptide(s) or polypeptide(s) or protein(s) of the invention comprising an HCV NS5 protein or part or derivative of any thereof modified as described herein. Said plurality of antigens may comprise a combination of HCV NS5 proteins or parts thereof or derivatives of any thereof derived from different HCV genotypes and/or subtypes and/or isolates.

A "vaccine composition" is an immunogenic composition capable of eliciting an immune response sufficiently broad and vigorous to provoke one or both of:

- a stabilizing effect on the multiplication of a pathogen already present in a host and against which the vaccine composition is targeted; and
- an effect increasing the rate at which a pathogen newly introduced in a host, after immunization with a vaccine composition targeted against said pathogen, is resolved from said host.

A vaccine composition may also provoke an immune response broad and strong enough to exert a negative effect on the survival of a pathogen already present in a host or broad and strong enough to prevent an immunized host from developing disease symptoms caused by a newly introduced pathogen. In particular the vaccine composition of the invention is a HCV vaccine composition wherein the pathogen is HCV.

An "effective amount" of an antigen in a vaccine composition is referred to as an amount of antigen required and sufficient to elicit an immune response. It will be clear to the skilled artisan that the immune response sufficiently broad and vigorous to provoke the effects envisaged by the vaccine composition may require successive (in time) immunizations with the vaccine composition as part of a vaccination scheme or vaccination schedule. The "effective amount" may vary depending on the health and physical condition of the individual to be treated, the taxonomic group of the individual to be treated (e.g. human, non-human primate, primate, etc.), the capacity of the individual's immune system to mount an effective immune response, the degree of protection desired, the formulation of the vaccine, the treating doctor's assessment, the strain of the infecting pathogen and other relevant factors. It is expected that the amount will fall in a relatively broad range that can be determined through routine trials. Usually, the amount will vary from 0.01 to 1000 μg/dose, more particularly from 0.1 to 100 μg/dose. Dosage treatment may be a single dose schedule or a multiple dose schedule. The vaccine may be administered in conjunction with other immunoregulatory agents.

A "prophylactic vaccine composition" is a vaccine composition providing protective immunity, i.e., an immunity preventing development of disease upon challenge of the host immunized with the prophylactic vaccine composition. In particular for HCV, a prophylactic HCV vaccine composition is to be understood as a vaccine composition capable of providing protective immunity helping to resolve a challenge HCV infection rapidly and/or preventing a challenge HCV infection to proceed to a chronic infection. Accelerated HCV viral clearance or accelerated control of HCV challenge infection is thus envisaged by vaccination with a prophylactic HCV composition according to the invention.

A "prophylactically effective amount" of an antigen in a prophylactic vaccine composition is referred to as an amount of antigen required and sufficient to elicit an immune response enabling the development of protective immunity. It will be clear to the skilled artisan that the immune response sufficiently broad and vigorous to provoke the effects envisaged by the prophylactic vaccine composition may need require successive (in time) immunizations with the prophylactic vaccine composition (see also "effective amount").

A "therapeutic vaccine composition" is a vaccine composition providing a curative immune response, i.e., an immune response capable of effectuating a reversion, or at least capable of effectuating halting, of disease symptoms associated with an already established pathogen infection. In particular for HCV, a therapeutic HCV vaccine composition is to be understood as a vaccine compositions capable of reducing serum liver enzyme, e.g., alanine aminotransferase (ALT) or γ-glutamylpeptidase (γ-GT), activity levels in the blood and/or of reducing HCV RNA levels and/or of reducing liver disease and/or of reducing liver fibrosis and/or of reducing liver fibrosis progression and/or reducing HCV antigen levels in or presented on liver cells.

A "therapeutically effective amount" of an antigen in a therapeutic vaccine composition is referred to as an amount of antigen required and sufficient to elicit an immune response enabling the development of a curative immune response. It will be clear to the skilled artisan that the antigenic or immunogenic response sufficiently broad and vigorous to provoke the effects envisaged by the therapeutic vaccine composition may need require successive (in time) immunizations with the therapeutic vaccine composition (see also "effective amount").

The HCV immunogenic composition, HCV vaccine composition, prophylactic HCV vaccine composition and/or therapeutic HCV vaccine composition comprises an HCV NS5 protein or part or a derivative of any thereof modified as described herein.

Another aspect of the current invention relates to the use of an isolated protein or part thereof or derivative of any thereof according to the invention for the manufacture of a HCV immunogenic composition, a prophylactic HCV vaccine composition or a therapeutic HCV vaccine composition.

Further aspects of the current invention comprise the HCV immunogenic composition, an HCV vaccine composition, a prophylactic HCV vaccine composition and/or a therapeutic HCV vaccine composition according to the invention for; or alternatively comprises the use of said composition for:

- inducing in a mammal a humoral response to the HCV peptides comprised in any of said compositions; and/or
- inducing in a mammal a cellular response to the HCV peptides comprised in any of said compositions, wherein said cellular response may be a $CD4^+$ T-cell proliferation response and/or a $CD8^+$ cytotoxic T-cell response and/or the increased production of cytokines; and/or
- prophylactic protection of a mammal against chronic HCV infection, wherein said HCV infection may be a homologous or a heterologous HCV infection; and/or
- therapeutically treating a chronically HCV-infected mammal, wherein said HCV may be a homologous or a heterologous HCV; and/or
- reducing liver disease in a HCV-infected mammal; and/or
- reducing liver disease in a chronic HCV-infected mammal by at least 2 points according to the overall Ishak score; and/or
- reducing serum liver enzyme activity levels in a HCV-infected mammal, wherein said liver enzyme may be, e.g., alanine aminotransferase (ALT) or gamma-glutamylpeptidase; and/or
- reducing HCV RNA levels in a HCV-infected mammal; and/or
- reducing liver fibrosis progression in a HCV-infected mammal; and/or
- reducing liver fibrosis in a HCV-infected mammal; and/or
- reducing HCV antigen levels in or presented on liver cells, wherein said HCV antigens include E2 or Core antigens.

Said mammal obviously may be a human. In particular, the uses according to the invention are methods for obtaining at least one of the recited effects, with said methods comprising administering any of said compositions to a mammal or a human. The recited effects may be obtained in combination with a DNA vaccine or with a DNA vector or DNA vaccine vector capable of expressing or effectuating expression of one or more antigens. A DNA vaccine, DNA vector or DNA vaccine vector may be a HCV DNA vaccine, HCV DNA vector or HCV DNA vaccine vector (see further).

With "prophylactic protection against infection by a homologous HCV" is meant that protection is obtained against a challenge HCV virus of exactly the same genotype, subtype or isolate as compared to the HCV genotype, subtype or isolate from which the HCV antigen or HCV antigens are derived. A composition may for example comprise a peptide or polypeptide according to the present invention that is derived from a particular HCV type 1b isolate. A "homologous HCV" would in this case be the same particular HCV type 1b isolate. "Homologous" in the context of "therapeutic treatment of a HCV homologous to the HCV peptides in a composition" has to be interpreted likewise.

With "prophylactic protection against infection by a heterologous HCV" is meant that protection is obtained against a challenge HCV virus classified in another genotype, subtype, or isolate as compared to the HCV genotype, subtype or isolate from which the HCV antigen or HCV antigens are derived. A composition may for example comprise a peptide or polypeptide according to the present invention that is derived from a particular HCV type 1b isolate. A "heterologous HCV" would in this case be, e.g., a HCV type 1b isolate sufficiently different from the type 1b isolate from which the antigens were derived, a type 1a HCV virus or a type 7 HCV virus. "Sufficiently different" as used in this particular context is to be understood at least a difference of 2%, 3% or 4% on the amino acid level. "Heterologous" in the context of "therapeutic treatment of a HCV heterologous to the HCV peptides in a composition" has to be interpreted likewise.

With the term "liver disease" is meant in this context any abnormal liver condition caused by infection with the hepatitis C virus including steatosis, inflammation, fibrosis, cirrhosis, necrosis, necro-inflammation and hepatocellular carcinoma.

With "reducing liver disease" is meant any stabilization or reduction of the liver disease status. Liver disease can be determined, e.g., by the Knodell scoring system (Knodell et al. 1981) or the Knodell scoring system adapted by Ishak (Ishak et al. 1995). A reduction of this score by two points is accepted as therapeutically beneficial effect in several studies (see, e.g., studies published after 1996 as indicated in Table 2 of Shiffman 1999).

With "reducing liver fibrosis progression" is meant any slowing down, halting or reverting of the normally expected progression of liver fibrosis. Liver fibrosis progression can be determined, e.g., by the Metavir scoring system. Normal expected progression of liver fibrosis according to this system was published to be an increase of the Metavir score of an untreated chronic HCV patient of approximately 0.133 per year (Poynard et al. 1997). "Reducing liver fibrosis" is meant to comprise any reduction of the normally expected progression of liver fibrosis.

Liver fibrosis and inflammation can be scored according to the Ishak scoring system (which is a modification of the scoring system of Knodell et al. 1981; Ishak et al. 1995) or Metavir scoring system (Bedossa and Poynard 1996). The Ishak scores range from 0 to 18 for grading of inflammation and from 0 to 6 for staging of fibrosis/cirrhosis. The sum of the Ishak inflammation and fibrosis scores comes closest to the Histological Activity Index (HAI; Knodell et al. 1981) which has been widely used. The Metavir scores range from 0 to 3 for grading of inflammation and from 0 to 4 for staging of fibrosis/cirrhosis. The overall progression rate of the Metavir score in an untreated patient is estimated to be 0.133 per year (Poynard et al. 1997).

Currently known HCV types include HCV genotypes 1, 2, 3, 4, 5, 6, 7, 8, 9, 10, 11 and known subtypes thereof include HCV subtypes 1a, 1b, 1c, 1d, 1e, 1f, 1g, 2a, 2b, 2c, 2d, 2e, 2f, 2g, 2h, 2i, 2k, 21, 3a, 3b, 3c, 3d, 3e, 3f, 3g, 4a, 4b, 4c, 4d, 4e, 4f, 4g, 4h, 4i, 4j, 4k, 41, 4m, 5a, 6a, 6b, 7a, 7b, 7c, 7d, 8a, 8b, 8c, 8d, 9a, 9b, 9c, 10a and 11a. The sequences of cDNA clones covering the complete genome of several prototype isolates have been determined and include complete prototype genomes of the HCV genotypes 1a (e.g., GenBank accession number AF009606), 1b (e.g., GenBank accession number AB016785), 1c (e.g., GenBank accession number D14853), 2a (e.g., GenBank accession number AB047639), 2b (e.g., GenBank accession number AB030907), 2c (e.g., GenBank accession number D50409) 2k (e.g., GenBank accession number AB031663), 3a (e.g., GenBank accession number AF046866), 3b (e.g., GenBank accession number D49374), 4a (e.g., GenBank accession number Y11604), 5a (e.g., GenBank accession number AF064490), 6a (e.g., GenBank accession number Y12083), 6b (e.g., GenBank accession number D84262), 7b (e.g., GenBank accession number D84263), 8b (e.g., GenBank accession number D84264), 9a (e.g., GenBank accession number D84265), 10a (e.g., GenBank accession number D63821) and 11a (e.g., GenBank accession number D63822). A new HCV genotype was further described in International Patent Publication No. WO03/020970. An HCV isolate is to be considered as a HCV quasispecies isolated from a HCV-infected mammal. A HCV quasispecies usually comprises a number of variant viruses with variant genomes usually of the same HCV type or HCV subtype.

A "pharmaceutically acceptable carrier" or "pharmaceutically acceptable adjuvant" is any suitable excipient, diluent, carrier and/or adjuvant which, by themselves, do not induce the production of antibodies harmful to the individual receiving the composition nor do they elicit protection. Preferably, a pharmaceutically acceptable carrier or adjuvant enhances the immune response elicited by an antigen. Suitable carriers or adjuvantia typically comprise one or more of the compounds included in the following non-exhaustive list:

- large slowly metabolized macromolecules such as proteins, polysaccharides, polylactic acids, polyglycolic acids, polymeric amino acids, amino acid copolymers and inactive virus particles;
- aluminium hydroxide, aluminium phosphate (see International Patent Application Publication No. WO93/24148), alum ($KA1(SO_4)_2.12H_2O$), or one of these in combination with 3-0-deacylated monophosphoryl lipid A (see International Patent Application Publication No. WO93/19780);
- N-acetyl-muramyl-L-threonyl-D-isoglutamine (see U.S. Patent No. 4,606,918), N-acetyl-normuramyl-L-alanyl-D-isoglutamine, N-acetylmuramyl-L-alanyl-D-isoglutamyl-L-alanine2-(1',2'-dipalmitoyl-sn-glycero-3-hydroxyphosphoryloxy) ethylamine;
- RIBI (ImmunoChem Research Inc., Hamilton, MT, USA) which contains monophosphoryl lipid A (i.e., a detoxified endotoxin), trehalose-6,6-dimycolate, and cell wall skeleton (MPL + TDM + CWS) in a 2% squalene/Tween 80 emulsion. Any of the three components MPL, TDM or CWS may also be used alone or combined 2 by 2. The MPL may also be replaced by its synthetic analogue referred to as RC-529 or by any other amino-alkyl glucosaminide 4-phosphate (Johnson et al. 1999, Persing et al. 2002);
- adjuvants such as Stimulon (Cambridge Bioscience, Worcester, MA, USA), SAF-1 (Syntex);
- bacterial DNA-based adjuvants such as ISS (Dynavax) or CpG (Coley Pharmaceuticals);
- adjuvants such as combinations between QS21 and 3-de-O-acetylated monophosphoryl lipid A (see International Patent Application Publication No. WO94/00153) which may be further supplemented with an oil-in-water emulsion (see, e.g., International Patent Application Publication Nos. WO95/17210, WO97/01640 and WO9856414) in which the oil-in-water emulsion comprises a metabolisable oil and a saponin, or a metabolisable oil, a saponin, and a sterol, or which may be further supplemented with a cytokine (see International Patent Application Publication No. WO98/57659);
- adjuvants such as MF-59 (Chiron), or poly[di(carboxylatophenoxy) phosphazene] based adjuvants (Virus Research Institute);
- blockcopolymer based adjuvants such as Optivax (Vaxcel, Cytrx) or inulin-based adjuvants, such as Algammulin and GammaInulin (Anutech);
- Complete or Incomplete Freund's Adjuvant (CFA or IFA, respectively) or Gerbu preparations (Gerbu Biotechnik). It is to be understood that Complete Freund's Adjuvant (CFA) may be used for non-human applications and research purposes as well;
- a saponin such as QuilA, a purified saponin such as QS21, QS7 or QS17, β-escin or digitonin;
- immunostimulatory oligonucleotides comprising unmethylated CpG dinucleotides such as [purine-purine-CG-pyrimidine-pyrimidine] oligonucleotides. Immunostimulatory oligonucleotides may also be combined with cationic peptides as described, e.g., by Riedl et al. (2002);
- Immune Stimulating Complexes together with saponins, for example Quil A (ISCOMS);
- excipients and diluents, which are inherently non-toxic and non-therapeutic, such as water, saline, glycerol, ethanol, wetting or emulsifying agents, pH buffering substances, preservatives, and the like;

- a biodegradable and/or biocompatible oil such as squalane, squalene, eicosane, tetratetracontane, glycerol, peanut oil, vegetable oil, in a concentration of, e.g., 1 to 10% or 2.5 to 5%;
- vitamins such as vitamin C (ascorbic acid or its salts or esters), vitamin E (tocopherol), or vitamin A;
- carotenoids, or natural or synthetic flavanoids;
- trace elements, such as selenium;
- any Toll-like receptor ligand as reviewed in Barton and Medzhitov (2002).

Any of the afore-mentioned adjuvants comprising 3-de-O-acetylated monophosphoryl lipid A, said 3-de-O-acetylated monophosphoryl lipid A may be forming a small particle (see International Patent Application Publication No. WO94/21292).

A "pharmaceutically acceptable vehicle" includes vehicles such as water, saline, physiological salt solutions, glycerol, ethanol, etc. Auxiliary substances such as wetting or emulsifying agents, pH buffering substances, preservatives may be included in such vehicles.

Typically, a vaccine composition is prepared as an injectable, either as a liquid solution or suspension. Injection may be subcutaneous, intramuscular, intravenous, intraperitoneal, intrathecal, intradermal, intraepidermal. Other types of administration comprise implantation, suppositories, oral ingestion, enteric application, inhalation, aerosolization or nasal spray or drops. Solid forms, suitable for dissolving in, or suspension in, liquid vehicles prior to injection may also be prepared. The preparation may also be emulsified or encapsulated in liposomes for enhancing adjuvant effect.

Other aspects of the invention relate to methods of vaccinating a HCV-naïve or HCV-infected mammal comprising administering an HCV immunogenic composition, an HCV vaccine composition, a prophylactic HCV vaccine composition and/or a therapeutic HCV vaccine composition according to the invention in combination with (i.e., before, after or concurrently with) administering a DNA vaccine.

The immunogenic composition, vaccine composition, therapeutic vaccine composition or prophylactic vaccine composition as described above may in addition comprise DNA vaccine vectors capable of expressing or effectuating expression of an antigen. Particularly relating to the current invention, the HCV immunogenic composition, HCV vaccine composition, therapeutic HCV vaccine composition or prophylactic HCV vaccine composition may in addition comprise DNA vaccine vectors capable of expressing or effectuating expression of one or more antigens such as HCV proteins or parts thereof, e.g., a HCV NS5 protein or part thereof. Alternatively, the protein- or peptide-based immunogenic composition, vaccine composition, therapeutic vaccine composition or prophylactic vaccine composition of the invention may be used in combination with a DNA vector-based immunogenic composition, vaccine composition, therapeutic vaccine composition or prophylactic vaccine composition (also referred to as "DNA vaccine" or "HCV DNA vaccine" if the DNA vector comprised therein is encoding a HCV protein or part thereof). Such combination for instance includes a DNA-prime protein-boost vaccination scheme wherein vaccination is initiated by administering a DNA vector-based immunogenic composition, vaccine composition, therapeutic vaccine composition or prophylactic vaccine composition and is followed by administering a protein- or peptide-based immunogenic composition, vaccine composition, therapeutic vaccine composition or prophylactic vaccine composition of the invention. In particular the DNA vaccine vector is capable of expressing one or more HCV antigens or proteins or parts thereof.

With a "DNA vector" or "DNA vaccine vector" is meant any DNA carrier comprising the open reading frame for one or more of the peptides useful for eliciting and/or enhancing an immune response. In general, said open reading frames are operably linked to transcription regulatory elements, such as promoters and terminators, enabling expression of the peptide encoded by the open reading frame. The terms "DNA vector" or "DNA vaccine vector" are meant to include naked plasmid DNA, plasmid DNA formulated with a suitable pharmaceutically acceptable carrier, recombinant viruses (e.g., as described above), or recombinant viruses formulated with a suitable pharmaceutically acceptable carrier. A "HCV DNA vector" or "HCV DNA vaccine vector" relates to any DNA carrier comprising the open reading frame for one or more of the HCV peptides.

As used herein, the term "transcription regulatory elements" refers to a nucleotide sequence which contains essential regulatory elements, such that upon introduction into a living vertebrate cell it is able to direct the cellular machinery to produce translation products encoded by the polynucleotide.

The term "operably linked" refers to a juxtaposition wherein the components are configured so as to perform their usual function. Thus, transcription regulatory elements operably linked to a nucleotide sequence are capable of effecting the expression of said nucleotide sequence. Those skilled in the art can appreciate that different transcriptional promoters, terminators, carrier vectors or specific gene sequences may be used successfully

Another aspect of the current invention relates to the use of an isolated protein or part thereof or derivative of any thereof according to the invention in immunoassays, to the incorporation of an isolated protein or part thereof or derivative of any thereof according to the invention in immunoassay kits or diagnostic kits, and to the use of an isolated protein or part thereof or derivative of any thereof according to the invention for the manufacture of a an immunoassay kit or diagnostic kit. Immunoassays comprise immunological methods for determining the presence of antibodies to HCV in a biological sample or of antigens of HCV in a biological sample or of HCV virus in a biological sample, or for diagnosing HCV infection. Diagnostic kits or immunoassay kits comprise kits for determining the presence of antibodies to HCV in a biological sample or of antigens of HCV in a biological sample or of HCV virus in a biological sample, or for diagnosing HCV infection.

In particular said biological sample is suspected to contain HCV antibodies, HCV antigens or HCV virus.

A first general embodiment in relation to immunoassays comprises a method for determining the presence of antibodies to HCV, in particular to HCV NS5, in a biological sample comprising the step of detecting said antibodies to an isolated protein or part thereof or derivative of any thereof according to the invention.

A second general embodiment in relation to immunoassays comprises a method for determining the presence of HCV NS5 antigens in a biological sample comprising the step of detecting said HCV NS5 antigens with an antibody to said HCV NS5 antigens in the presence of an isolated protein or part thereof or derivative of any thereof according to the invention as competitor of binding of said HCV NS5 antigens to said antibody.

In particular said immunoassays relying on an isolated HCV NS5 protein or part thereof or derivative of any thereof according to the invention wherein at least one cysteine is reversibly modified are performed in the absence of a reducing agent.

A first specific embodiment in relation to immunoassays comprises a method for determining the presence of antibodies to HCV, in particular to HCV NS5, in a biological sample comprising the steps of:

(i) contacting said biological sample with an isolated protein or part thereof or derivative of any thereof according to the invention;
(ii) detecting the immunological complex formed between said antibodies and said protein or part thereof or derivative of any thereof;

wherein if at least one cysteine thiol group of HCV NS5 in said isolated protein or part thereof or derivative of any thereof is reversibly modified steps (i) and (ii) are performed in the absence of a reducing agent.

A second specific embodiment in relation to immunoassays comprises a method for determining the presence of a HCV virus in a biological sample comprising the steps of:

(i) contacting said biological sample with an isolated protein or part thereof or derivative of any thereof according to the invention;
(ii) detecting the immunological complex formed between antibodies to said HCV virus present in said sample and said protein or part thereof or derivative of any thereof;
(iii) inferring from the immunological complex formed in (ii) the presence of a HCV virus in said biological sample;

wherein if at least one cysteine thiol group of HCV NS5 in said isolated protein or derivative thereof is reversibly modified steps (i) and (ii) are performed in the absence of a reducing agent.

A third specific embodiment in relation to immunoassays comprises a method for diagnosing HCV infection in a mammal comprising the steps of:

(i) contacting a biological sample from said mammal with an isolated protein or part thereof or derivative of any thereof according to the invention;
(ii) detecting the immunological complex formed between antibodies to HCV present in said sample and said protein or part thereof or derivative of any thereof;
(iii) diagnosing from the immunological complex formed in (ii) HCV infection in said mammal;

wherein if at least one cysteine thiol group of HCV NS5 in said isolated protein or derivative thereof is reversibly modified steps (i) and (ii) are performed in the absence of a reducing agent.

A forth specific embodiment in relation to immunoassays comprises a method for determining the presence of a HCV NS5 antigen in a biological sample comprising the steps of:

(i) contacting said biological sample with an antibody to said HCV NS5 antigen in the presence of an isolated protein or part thereof or derivative of any thereof according to the invention as competitor, i.e. as competitor of binding of said HCV NS5 antigen to said antibody;
(ii) inferring from the immunological complex formed between said antibodies and said HCV NS5 antigen the presence of said HCV NS5 antigen;

wherein if at least one cysteine thiol group of HCV NS5 in said isolated protein or part thereof or derivative of any thereof is reversibly modified steps (i) and (ii) are performed in the absence of a reducing agent.

A fifth specific embodiment in relation to immunoassays comprises a method for determining the presence of a HCV virus in a biological sample comprising the steps of:

(i) contacting said biological sample with an antibody to an HCV NS5 antigen in the presence of an isolated protein or part thereof or derivative of any thereof according to the invention as competitor;
(ii) detecting the immunological complex formed between said antibodies and said HCV NS5 antigen;
(iii) inferring from the immunological complex formed in (ii) the presence of a HCV virus in said biological sample;

wherein if at least one cysteine thiol group of HCV NS5 in said isolated protein or part thereof or derivative of any thereof is reversibly modified steps (i) and (ii) are performed in the absence of a reducing agent.

A sixth specific embodiment in relation to immunoassays comprises a method for diagnosing HCV infection in a mammal comprising the steps of:

(i) contacting a biological sample from said mammal with an antibody to an HCV NS5 antigen in the presence of an isolated protein or part thereof or derivative of any thereof according to the invention as competitor;
(ii) detecting the immunological complex formed between said antibodies and said HCV NS5 antigen;
(iii) diagnosing from the immunological complex formed in (ii) HCV infection in said mammal;

wherein if at least one cysteine thiol group of HCV NS5 in said isolated protein or part thereof or derivative of any thereof is reversibly modified steps (i) and (ii) are performed in the absence of a reducing agent.

A further embodiment relates to the use of a protein or part thereof or derivative of any thereof according to the invention in an immunoassay.

Yet another embodiment relates to the use of a protein or part thereof or derivative of any thereof according to the invention in the manufacture of an immunoassay or immunoassay kit.

A further embodiment relates to a diagnostic kit for determining the presence of antibodies to HCV (in particular to HCV NS5) in a biological sample, for determining the presence of HCV NS5 antigens, in a biological sample, for determining the presence of a HCV virus in a biological sample or for diagnosing HCV infection in a mammal, said kit comprising an isolated protein or part thereof or derivative of any thereof according to the invention.

The proteins or parts thereof or derivatives of any thereof according to the present invention may be employed in virtually any immunoassay format that employs a known antigen to detect antibodies or a known antibody to detect antigens. A common feature of all of these assays is that the antigen is contacted with the body component containing or suspected of containing HCV antibodies or HCV antigens under conditions that permit binding between an antigen and an antibody, i.e. under conditions allowing the formation of an immunological complex. Such conditions will typically be physiologic temperature, pH and ionic strength using an excess of antigen (in the case of antibody detection) or antibody (in the case of antigen detection). The incubation of the antigen or antibody with the specimen is followed by detection of immune complexes.

The design of immunoassays is subject to a great deal of variation, and many formats are known in the art. Protocols may, for example, use solid supports, or immunoprecipitation. Most assays involve the use of labeled antibody and/or labeled polypeptide, e.g. a labeled peptide or polypeptide according to the present invention; the labels may be, for example, enzymatic, fluorescent, chemiluminescent, radioactive, or dye molecules. Assays which amplify the signals from the immune complex are also known; examples of which are assays which utilize biotin and avidin or streptavidin, and enzyme-labeled and mediated immunoassays, such as ELISA and RIA assays. Other immunoassay designs comprise line immunoassays, sandwich immunoassays, antigen down immunoassays. An immunoassays may be set up in a competitive format.

An immunoassay may be, without limitation, in a heterogeneous or in a homogeneous format, and of a standard or competitive type. In a heterogeneous format, the polypeptide is typically bound to a solid matrix, solid support or solid phase to facilitate separation of the sample from the polypeptide after incubation. Examples of solid supports, matrices or phases are listed furtheron. The solid support containing the antigenic polypeptides is typically washed after separating it from the test sample, and prior to detection of bound antibodies. Both standard and competitive formats are know in the art.

In a homogeneous format, the test sample is incubated with the combination of antigens in solution. For example, it may be under conditions that will precipitate any antigen-antibody complexes which are formed. Both standard and competitive formats for these assays are known in the art.

In a standard format, the amount of antibodies, such as anti-HCV antibodies, in the antibody-antigen complexes is directly monitored. This may be accomplished by determining whether labeled anti-xenogeneic (e.g. anti-human) antibodies which recognize an epitope on said antibodies, such as said anti-HCV antibodies, will bind due to complex formation. In a competitive format, the amount of said antibodies, such as said anti-HCV antibodies, in a sample is deduced by monitoring the competitive effect on the binding of a known amount of (labeled) antibody (or other competing ligand) or antigen in the complex.

Antigen-antibody complexes can be detected by any of a number of known techniques, depending on the format. For example, unlabeled antibodies such as anti-HCV antibodies in the complex may be detected using a conjugate of anti-xenogeneic Ig complexed with a label (e.g. an enzyme label).

In an immunoprecipitation or agglutination assay format the reaction between an antigen and an antibody forms a protein cluster that precipitates from the solution or suspension and forms a visible layer or film of precipitate. If no antibody is present in the test specimen or sample, no such precipitate is formed.

A diagnostic kit usually comprises a molecule for detecting the presence of a sample reactant capable of interacting with said molecule, of a sample reactant modifying said molecule (e.g., in a chemical reaction), and/or of a sample reactant modifiable by said molecule (e.g., in a chemical reaction). In a diagnostic kit for detection of an antigen or antibody in a sample, one or more antibodies or antigens, respectively, are part of said kit. In a diagnostic kit for detecting antigens or antibodies, antibodies or antigens, respectively, are often present on a solid phase, matrix or support.

The proteins or parts thereof or derivatives of any thereof according to the present invention can be packaged and be part of a diagnostic kit. The kit will normally contain in separate containers or vials the peptides or polypeptides according to the present invention (labelled or unlabelled), control antibody formulations (positive and/or negative), labelled antibody when the assay format requires the same and signal generating reagents (e.g. enzyme substrate) if the label does not generate a signal directly. The peptides or polypeptides according to the present invention may be already bound to a solid matrix or may be present in the kit in a separate vial together with reagents for binding it to the matrix. Instructions (e.g. written, tape, CD-ROM, etc.) for carrying out the assay usually will be included in the kit.

The signal generating compound can include an enzyme, a luminescent compound, a chromogen, a radioactive element and a chemiluminescent compound. Examples of enzymes include alkaline phosphatase, horseradish peroxidase and beta-galactosidase. Examples of enhancer compounds include biotin, anti-biotin and avidin. Examples of enhancer compounds binding members include biotin, anti-biotin and avidin. In order to block the effects of rheumatoid factor-like substances, the test sample is subjected to conditions sufficient to block the effect of rheumatoid factor-like substances. These conditions comprise contacting the test sample with a quantity of anti-human IgG to form a mixture, and incubating the mixture for a time and under conditions sufficient to form a reaction mixture product substantially free of rheumatoid factor-like substance.

Solid phases, solid matrices or solid supports on which molecules, e.g., the antigens of the present invention, may be bound (or captured, absorbed, adsorbed, linked, coated, immobilized; covalently or non-covalently) comprise beads or the wells or cups of microtiter plates, or may be in other forms, such as solid or hollow rods or pipettes, particles, e.g., from 0.1 µm to 5 mm in diameter (e.g. "latex" particles, protein particles, or any other synthetic or natural particulate material), microspheres or beads (e.g. protein A beads, magnetic beads). A solid phase may be of a plastic or polymeric material such as nitrocellulose, polyvinyl chloride, polystyrene, polyamide, polyvinylidine fluoride or other synthetic polymers. Other solid phases include membranes, sheets, strips, films and coatings of any porous, fibrous or bibulous material such as nylon, polyvinyl chloride or another synthetic polymer, a natural polymer (or a derivative thereof) such as cellulose (or a derivative thereof such as cellulose acetate or nitrocellulose). Fibers or slides of glass, fused silica or quartz are other examples of solid supports. Paper, e.g., diazotized paper may also be applied as solid phase. Clearly, molecules, *in casu* the antigens of the present invention, may be bound, captured, absorbed, adsorbed, linked or coated to any solid phase suitable for use in immunoassays. Said molecules, *in casu* the antigens of the present invention, may be present on a solid phase in defined zones such as spots or lines.

Any of the above described solid phases may be developed, e.g. automatically developed in an assay device.

With "developed" or "development" is meant that a sample or samples, suspected of comprising a binding partner to a molecule present on a solid phase, is or are applied to said solid phase and that the necessary steps are performed in order to detect binding of the binding partner to a molecule on a solid phase. This can, e.g., be the detection of binding of an antibody suspected to be present in a biological sample to an antigen, *in casu* an antigen of the present invention, present on a solid phase. Automatic development hence refers to a development process, or any one or more steps thereof, in an automated or robotized fashion. A development automate or robot (or, generally, an assay device) generally is connected to or comprises one, more or all of the development or assay reagents and may in addition comprise a means to "read" the developed assay. Said "reading" will logically depend on the assay and may, e.g., confer to determining color intensities, to determining optical density or absorption at a given wavelength, to determining fluoresence, fosforescence or (chemi)luminescence, to determining turbidity, to determining the decay of a radio-active element or to determining other physical or physico-chemical characteristics that are related to the binding of a binding partner in a sample to a molecule present on a solid phase.

A biological sample may be a liquid test sample or a solid test sample. A liquid test sample may be any body fluid, for example, blood, plasma, serum, saliva, urine, cerebro-spinal fluid, milk, lymph fluid, tears, or secretions of the respiratory, intestinal or genito-urinary tracts. A solid test sample such as cells or tissue may be brought into liquid form for testing, for example, as tissue exudate or macerate. A solid test sample such as cells or tissue may be fixed, or fixed and sectioned, an example thereof being formalin-fixed paraffin-embedded liver tissue sections.

## EXAMPLES

### EXAMPLE 1. Production of NS3 in *Escherichia coli.*

As an example of HCV NS3 protein production, production of the HCV NS3-TN protein is herein given. This production method can, however, be applied to other HCV NS3 proteins (or fragments thereof) as well. The HCV NS3-TN protein (amino acids 1166-1468 of the HCV polyprotein in which the amino acids 1167 to 1180 have been replaced by the amino acids 1071-1084, as described in Example 7a of International Patent Application No. PCT/EP99/04342 (Publication No. WO 99/67285)) was expressed in *E. coli.*

The NS3-TN protein (SEQ ID NO:1) was purified essentially as described in Example 7b of International Patent Application No. PCT/EP99/04342 (Publication No. WO 99/67285) making use of sulfonation as modifying agent for the cysteines, thus yielding sulfonated NS3-TN (NS3-TN SO3).

Alternatively, cysteine thiol-groups in the NS3-TN protein were blocked by means of alkylation with iodoacetamide. Thereto, NS3-TN SO3 was incubated in 50 mM DTT for 30 minutes at 37°C followed by an alkylation step in which iodoacetamide was added to a final concentration of 200 mM (30 minutes at 37°C). This yielded the alkylated NS3-TN (NS3-TN IAA).

Finally the NS3-TN SO3 and NS3-TN IAA material was desalted to PBS, pH 7.5 containing 6 M urea. NS3-TN SO3 was thus obtained at 1.45 mg/mL, and NS3-TN IAA at 1.9 mg/mL.

### EXAMPLE 2. Production of NS5A in *Escherichia coli.*

An *E. coli* JM83 strain transformed with the plasmid pIGFH113NS5a plasmid (Figure 1; nucleotide sequence of plasmid given in SEQ ID NO:2) was used for production of an exemplary mTNF-H6-NS5A protein as defined by SEQ ID NO:3. Plasmid pIGFH113NS5a was derived from vector pIGFH113 in which the HCV NS5a fragment was cloned. The HCV NS5a fragment was amplified by RT-PCR with primers HCPr5065 (SEQ ID NO:5) and HCPr5066 (SEQ ID NO:6) on serum sample No. 20020.

The mTNF-H6-NS5A protein (SEQ ID NO:3) is a fusion between an amino-terminal 26-amino acid murine TNF peptide, followed by a hexahistidine tag, followed by a 3-amino acid linker peptide, and followed by a HCV genotype 1b NS5A protein (spanning amino acids 1973 to 2420 of the HCV polyprotein; defined by SEQ ID NO:4).

An overnight grown seed-culture (2 x 0.5 L; in LB-medium supplemented with 10 mg/L tetracycline) was used to inoculate a 15-L fermentor (10% v/v inoculation). Expression of the mTNF-H6-NS5A protein was induced by addition of 0.1 mM IPTG from the start of the fermentation onwards. The fermentation medium NM (5 g/L yeast extract; 7.6 g/L $K_2HPO_4$; 3.5 g/L $KH_2PO_4$; 3.5 g/L $(NH_4)_2HPO_4$; 2 mL/15 L polypropyleneglycol) was used. During fermentation pH was maintained at 6.9 by the addition of 2.5% $NH_4OH$ solution or 8.5 % $H_3PO_4$ solution. The dissolved oxygen concentration was maintained at 20% of air-saturation. The fermentation temperature was maintained at 28°C throughout the fermentation. The culture was harvested after three hours. The culture broth was first 10-fold concentrated by tangential flow filtration (0.2 µm microfiltration) and cells were subsequently separated by a centrifugation step. The resulting cell pellets were stored at -70°C.

Cell pellets were resuspended [5 mL buffer/g cell pellet (wet weight)] in lysis buffer (50 mM Tris/HCl buffer, pH 8.0, to which 2 mM $MgCl_2$, 2 mM Pefabloc, 25 mM 6-$NH_2$-n-hexanoic acid and 10 U/mL benzonase grade II was added). After resuspension, cell disruption was performed by high-pressure homogenisation (1 pass at 1.4 kbar). The lysate was clarified by centrifugation (26000 x *g* for 1 hour at 4° C).

After addition of solid Gu.HCl (final concentration of 5.7 M) to the obtained supernatant, the protein was sulfonated overnight (at room temperature and pH 7.5) in the presence of 320 mM (4% w/v) sodium sulfite, 65 mM (2% w/v) sodium tetrathionate and 0.1 mM $CuSO_4$. After a freeze-thaw cycle, a centrifugation (26000 x g for 1 hour at 4° C) was performed and the supernatant was recovered.

A first purification step of the expressed and Gu.HCl-solubilized product was performed on Ni-IDA. *n*-Dodecyl-N,N-dimethylglycine (known under the trade name Empigen BB; Albright & Wilson) and imidazole were added to a final concentration of 1% (w/v) and 20 mM respectively and the pH was adjusted to pH 7.2. All further chromatographic steps were executed on an Akta FPLC workstation (Pharmacia). The sample was filtrated through a 0.22 µm pore size membrane (cellulose acetate) and loaded on a Ni-IDA column (Chelating Sepharose FF loaded with $Ni^{2+}$, Pharmacia), which was equilibrated with 50 mM phosphate, 6 M Gu.HCl, 1 % *n*-dodecyl-N,N-dimethylglycine, pH 7.2 (IMAC-buffer A) supplemented with 20 mM imidazole. The column was washed sequentially with IMAC-buffer A containing 20 mM, 35 mM and 50 mM imidazole respectively till the absorbance at 280 nm reached the baseline level. A buffer exchange was performed on column by a washing step with 25 mM Tris/HCl, 6 M urea, 0.1 % PF-127 at pH 7.2 (IMAC-buffer C). Further elution was performed sequentially by applying IMAC-buffer C containing first 100 mM and further 200 mM imidazole respectively. SDS-PAGE and 2 western-blot analyses [using a specific monoclonal antibody directed against hTNF (Innogenetics IGH104) and a Rb anti-*E. coli* antiserum (DAKO) respectively] of the different washing and elution fractions showed that the main part of the desired product was present in the 50 mM imidazole wash and 200 mM imidazole elution pools.

To remove some degradation and host contaminant products, a further fractionation by anion exchange chromatography was performed. The obtained 200 mM imidazole IMAC-elution pool was 2 x diluted with 20 mM Tris/HCl, 6 M urea, 0.05 % PF-127, 1 mM EDTA at pH 8.0 (Q-buffer A) and the pH was further adjusted to pH 8.0. The sample was loaded on an anion exchange column (POROS 20 QE, PerSeptive Biosystems), which was equilibrated with Q-buffer A. The chromatography protocol existed of a washing procedure over 5 column volumes with 90 % Q-buffer A and 10 % Q-buffer B (20 mM Tris/HCl, 6 M urea, 1 mM EDTA, 1M NaCl at pH 8.0), followed by a continuous gradient elution over 20 column volumes from 10 % Q-buffer B to 55 % Q-buffer B and a second continuous gradient elution over 10 column volumes from 55 % Q-buffer B to 100 % Q-buffer B. Elution fractions containing mainly pure and intact mTNF-H6-NS5A protein were selected based on SDS-PAGE and western blot analysis (as described above) and pooled.

A final purification step was performed by subjecting the obtained pool to the same chromatographic anion exchange procedure after a 4-fold dilution with Q-buffer A. Elution fractions containing the intact protein (based on SDS-PAGE and Westen-blot analysis of the different elution fractions; as described above) were pooled and concentrated by ultrafiltration (MWCO 10 kDa, centriplus, Amicon, Millipore). This yielded the mTNF-H6-NS5A wherein the cysteine thiol-groups were blocked by sulfonation (mTNF-H6-NS5A SO3) at a concentration of approximately 1.5 mg/mL.

Alternatively, cysteine thiol-groups in the mTNF-H6-NS5A protein were blocked by means of alkylation with iodoacetamide. Thereto, mTNF-H6-NS5A was incubated in 5 mM DTT for 30 minutes at 37°C followed by an alkylation step in which iodoacetamide was added to a final concentration of 15 mM (30 minutes at 37°C). This yielded the alkylated mTNF-H6-NS5A (mTNF-H6-NS5A IAA).

Finally the mTNF-H6-NS5A SO3 and mTNF-H6-NSSA IAA material was desalted on a Fast Desalting Column HR 10/10 (Pharmacia) to PBS, pH 7.0 containing 6 M urea. mTNF-H6-NS5A SO3 was thus obtained at 1.7 mg/mL, and mTNF-H6-NS5A IAA at 1.6 mg/mL. SDS-PAGE and 2 western-blot analyses (using a specific monoclonal antibody directed against hTNF (Innogenetics IGH104) and a Rb anti-*E. coli* antiserum (DAKO) respectively) of the final product indicated a purity of > 90 %.

**EXAMPLE 3. Antigenicity study of NS3 in ELISA.**

The sulfonated and alkylated NS3 batches from Example 1 were compared by ELISA with serum samples derived from HCV carriers or healthy donors. The sulfonated NS3 was analyzed as such but also after desulfonation. Coating was at 3 µg/ml in PBS, and for desulfonation 5 mM DTT was added to the coating buffer. The results are shown in Table 1. Based on the average reactivity shown at the bottom of the table in gray shading, both the alkylated and sulfonated (with or without DTT) have a very low reactivity with sera from healthy donors. There is however, a clear need for the sulfonated protein to be treated with DTT to improve the response with serum from HCV carriers. In the case of serum 17805 this sample would have been wrongly interpreted as negative for HCV antibodies if the sulfon groups would not have been removed. Surprisingly the alkyl groups interfere far less with detection of antibodies and the average reactivity of this protein is very similar to the reactivity of the desulfonated protein.

**EXAMPLE 4. Antigenicity study of NS5A in ELISA.**

The sulfonated and alkylated NS5A batches from Example 2 were compared with in ELISA with serum samples derived from HCV carriers or healthy donors. The sulfonated NS5A was analyzed as such but also after desulfonation. Coating was at 1 µg/ml in PBS, and for desulfonation 5 mM DTT was added to the coating buffer. The results are shown in Table 2. Based on the average reactivity, shown at the bottom of the table in gray shading, both the alkylated and sulfonated (with or without DTT) have a very low reactivity with sera from healthy donors. Surprisingly, there is in this case no need for the sulfonated protein to be treated with DTT to improve the response with serum from HCV carriers. On the contrary DTT treatment seems to reduce the reactivity which is even abolished in the case of serum 17786. Surprisingly these results are not identical compared to those for NS3 (see Example 3).

**EXAMPLE 5. Immunogenicity of NS5 in mice.**

The mTFN-H6-NS5A SO3 and mTFN-H6-NS5A IAA proteins obtained as described in Example 2 were diluted to 500 µg/mL with 0.9% NaCl, mixed with an equal volume of Alhydrogel 1.3% (Superfos, Denmark) and finally further diluted with 8 volumes of 0.9% NaCl to yield alum-adjuvanted NS5A at a concentration of 50 µg NS5A/mL and 0.13% of Alhydrogel.

Groups of 6 Balb/c mice were immunized intramuscularly three times with a three-week interval with 5 µg of either sulfonated or alkylated NS5A. The immune response was assessed 2 weeks after the third immunization.

ANTIBODY TITERS

Antibody titers were determined by ELISA. After coating with the specific (desulfonated with 5mM DTT for 1hr at 37°C) antigen form (3 µg/mL, overnight at 4°C) and blocking, serum was incubated in sample diluent. Highest (starting) serum concentration was 1/1000, and this concentration was further diluted, each time with 0.5 log 10 (or 1/3.16). As a conjugate, HRP labelled rabbit anti-mouse Ig (1/20 000, stock concentration of 1.3 mg/mL, DAKO) was used. For each titration, the log EC 50 values were calculated by Prism using following parameters: non-linear regression, sigmoidal dose-response curve with fixed bottom value (= blank). The results are summarized in Figure 2.

All animals mounted an antibody response against NS5A. Both the alkylated and the sulfonated protein induce a significant antibody response which can be detected both by alkylated or desulfonated protein in the ELISA. In none of the assays major differences could be detected indicating that both the sulfonated and alkylated NS5A protein induce antibody responses of a similar level and that cross-reactivity in ELISA versus alkylated or desulfon ated NS5A is also very similar.

T-CELL IMMUNITY

After isolation and counting, mice spleen cells were plated out at a concentration of 200 000 cells per well in flat bottom 96 well plates and were restimulated in vitro with each of the two different antigens at a final concentration of 1 µg/mL or with medium without any antigen added as control. After 5 days of culturing, $^3$H-thymidine (1 µCi/well) was incorporated overnight and cells were harvested the next morning. All experiments were performed in five fold. The results in figure are expressed as stimulation index (SI) . The SI is calculated with the following formula:

$$\frac{\text{mean cpm of 5 cultures stimulated with antigen}}{\text{mean cmp of 5 cultures stimulated without antigen}}$$

The mean SI, as can be judged from Figure 3 tends to be very similar for immunization with alkylated or sulfonated material and this irrespective of the NS5A protein used for in vitro restimulation.

**Table 1:** OD values as obtained in ELISA with sera from HCV carriers or healthy donors. The sera were incubated at a dilution of 1/20 on the NS3 coated plates. Ser nr = serum number. Avg = average.

| HCV-sera Ser nr | NS3 IAA | NS3 SO3 | NS3 SO3 +DTT | Healthy donor sera Ser nr | NS3 IAA | NS3 SO3 | NS3 SO3 +DTT |
|---|---|---|---|---|---|---|---|
| 17807 | 1.518 | 1.011 | 1.595 | F504 | 0.068 | 0.064 | 0.051 |
| 17842 | 1.522 | 0.292 | 1.569 | F511 | 0.062 | 0.063 | 0.050 |
| 17777 | 1.588 | 1.416 | 1.547 | F516 | 0.058 | 0.061 | 0.048 |
| 17785 | 1.579 | 1.421 | 1.550 | F517 | 0.061 | 0.058 | 0.053 |
| 17794 | 1.444 | 1.220 | 1.396 | F518 | 0.070 | 0.095 | 0.091 |
| 17798 | 1.149 | 0.944 | 1.433 | F519 | 0.143 | 0.134 | 0.110 |
| 17805 | 1.101 | 0.118 | 1.525 | F520 | 0.128 | 0.142 | 0.073 |
| 17810 | 1.698 | 1.267 | 1.706 | F521 | 0.114 | 0.143 | 0.088 |
| 17819 | 1.756 | 1.472 | 1.582 | F522 | 0.071 | 0.073 | 0.062 |
| 17826 | 1.574 | 1.208 | 1.544 | F523 | 0.095 | 0.189 | 0.125 |
| 17849 | 1.578 | 1.408 | 1.773 | F526 | 0.087 | 0.084 | 0.054 |
| 17763 | 1.596 | 1.427 | 1.701 | F529 | 0.086 | 0.085 | 0.079 |
| 17807 | 1.518 | 0.972 | 1.660 | F513 | 0.120 | 0.135 | 0.100 |
| 17808 | 1.455 | 1.156 | 1.639 | F530 | 0.064 | 0.067 | 0.050 |
| 17816 | 1.441 | 0.994 | 1.538 | F531 | 0.094 | 0.100 | 0.049 |
| 17820 | 0.346 | 0.304 | 1.013 | F527 | 0.098 | 0.092 | 0.074 |
| 55333 | 1.644 | 1.464 | 1.607 | F532 | 0.104 | 0.087 | 0.049 |
| 55337 | 1.625 | 1.211 | 1.403 | F533 | 0.089 | 0.108 | 0.079 |
| 55340 | 1.687 | 1.163 | 1.621 | F534 | 0.072 | 0.060 | 0.050 |
| 55342 | 1.723 | 1.251 | 1.561 | F535 | 0.067 | 0.077 | 0.055 |
| 55345 | 1.679 | 1.469 | 1.689 | F536 | 0.088 | 0.091 | 0.054 |
| 55348 | 1.436 | 0.987 | 1.526 | F552 | 0.072 | 0.073 | 0.065 |
| 55350 | 1.649 | 0.907 | 1.614 | F553 | 0.071 | 0.056 | 0.050 |
| 55352 | 1.341 | 0.905 | 1.427 | F555 | 0.066 | 0.063 | 0.044 |
| 55353 | 1.294 | 0.843 | 1.332 | avg | 0.085 | 0.092 | 0.067 |
| 55354 | 0.770 | 0.597 | 0.902 | | | | |
| 55355 | 1.306 | 0.911 | 1.315 | | | | |
| 55362 | 1.222 | 0.937 | 1.498 | | | | |
| 55365 | 1.396 | 1.365 | 1.364 | | | | |
| avg | 1.436 | 1.057 | 1.504 | | | | |

**Table 2:** OD values as obtained in ELISA with sera from HCV carriers or healthy donors. The sera were incubated at a dilution of 1/20 on the NS5 coated plates. Ser nr = serum number. Avg = average.

| HCV sera Ser nr | NS5 IAA | NS5 SO3 | NS5 SO3 +DTT | Healthy donor sera Ser nr | NS5 IAA | NS5 SO3 | NS5 SO3 +DTT |
|---|---|---|---|---|---|---|---|
| 17758 | 0,246 | 0,217 | 0,128 | F504 | 0,054 | 0,069 | 0,044 |
| 17767 | 1,630 | 1,487 | 1,523 | F511 | 0,045 | 0,063 | 0,049 |
| 17785 | 1,477 | 1,386 | 1,505 | F516 | 0,043 | 0,042 | 0,043 |
| 17786 | 0,508 | 0,510 | 0,127 | F517 | 0,043 | 0,050 | 0,043 |
| 17794 | 0,069 | 0,072 | 0,054 | F518 | 0,084 | 0,063 | 0,060 |
| 17798 | 1,533 | 1,461 | 1,383 | F519 | 0,160 | 0,139 | 0,063 |
| 17805 | 1,625 | 1,535 | 1,621 | F520 | 0,044 | 0,045 | 0,057 |
| 17807 | 1,476 | 1,567 | 1,549 | F521 | 0,043 | 0,043 | 0,041 |
| 17810 | 1,499 | 1,595 | 1,390 | F522 | 0,049 | 0,052 | 0,050 |
| 17819 | 1,401 | 1,482 | 1,456 | F523 | 0,044 | 0,044 | 0,041 |
| 17826 | 1,369 | 1,361 | 1,353 | F526 | 0,198 | 0,179 | 0,043 |
| 17849 | 1,548 | 1,476 | 1,451 | F529 | 0,044 | 0,044 | 0,044 |
| 17763 | 1,540 | 1,535 | 1,357 | F513 | 0,041 | 0,042 | 0,041 |
| 17807 | 1,452 | 1,517 | 1,446 | F530 | 0,053 | 0,051 | 0,043 |
| 17808 | 0,047 | 0,045 | 0,045 | F531 | 0,054 | 0,049 | 0,044 |
| 17816 | 1,167 | 1,254 | 0,957 | F527 | 0,161 | 0,163 | 0,158 |
| 17826 | 1,525 | 1,601 | 1,440 | F532 | 0,049 | 0,052 | 0,048 |
| 55333 | 1,602 | 1,655 | 1,437 | F533 | 0,127 | 0,104 | 0,040 |
| 55337 | 1,557 | 1,600 | 1,540 | F534 | 0,074 | 0,055 | 0,046 |
| 55340 | 1,604 | 1,582 | 1,554 | F535 | 0,043 | 0,042 | 0,042 |
| 55342 | 1,572 | 1,623 | 1,446 | F536 | 0,042 | 0,043 | 0,042 |
| 55348 | 1,471 | 1,489 | 1,165 | F552 | 0,042 | 0,042 | 0,043 |
| 55350 | 1,440 | 1,499 | 1,043 | F553 | 0,041 | 0,044 | 0,042 |
| 55352 | 1,568 | 1,614 | 1,248 | F555 | 0,044 | 0,043 | 0,043 |
| 55353 | 0,943 | 0,989 | 0,700 | F556 | 0,048 | 0,044 | 0,043 |
| 55354 | 0,952 | 0,942 | 0,649 | F557 | 0,043 | 0,043 | 0,045 |
| 55355 | 1,398 | 1,438 | 1,430 | F558 | 0,043 | 0,041 | 0,044 |
| 55368 | 0,046 | 0,066 | 0,043 | F559 | 0,043 | 0,047 | 0,053 |
| 55362 | 1,310 | 1,337 | 1,040 | avg | 0,064 | 0,062 | 0,050 |
| avg | 1,227 | 1,239 | 1,106 | | | | |

**REFERENCES**

1. Barton,G.M. & Medzhitov,R. Toll-like receptors and their ligands. *Curr. Top. Microbiol. Immunol.* **270,** 81-92 (2002).

2. Bedossa,P. & Poynard,T. An algorithm for the grading of activity in chronic hepatitis C. The METAVIR Cooperative Study Group. *Hepatology* **24,** 289-293 (1996).

3. Beekman,N.J. *et al.* Synthetic peptide vaccines: palmitoylation of peptide antigens by a thioester bond increases immunogenicity. *J. Pept. Res.* **50,** 357-364 (1997).

4. Bums,J., Butler,J. & Whitesides,G. Selective reduction of disulfides by Tris(2-carboxyethyl)phosphine. *J. Org. Chem.* **56,** 2648-2650 (1991).

5. Choo,Q.L. *et al.* Vaccination of chimpanzees against infection by the hepatitis C virus. *Proc. Natl. Acad. Sci. U. S. A* **91,** 1294-1298 (1994).

6. Darbre,A. Practical protein chemistry: a handbook. Whiley & Sons Ltd., (1986).

7. Forns,X. *et al.* DNA immunization of mice and macaques with plasmids encoding hepatitis C virus envelope E2 protein expressed intracellularly and on the cell surface. *Vaccine* **17**, 1992-2002 (1999).

8. Forns,X. *et al.* Vaccination of chimpanzees with plasmid DNA encoding the hepatitis C virus (HCV) envelope E2 protein modified the infection after challenge with homologous monoclonal HCV. *Hepatology* **32**, 618-625 (2000).

9. Fujihashi,K. *et al.* Cytokine-specific ELISPOT assay. Single cell analysis of IL-2, IL-4 and IL-6 producing cells. *J. Immunol. Methods* **160,** 181-189 (1993).

10. Gailit,J. Restoring free sulfhydryl groups in synthetic peptides. *Anal. Biochem.* **214,** 334-335 (1993).

11. Grakoui,A., Wychowski,C., Lin,C., Feinstone,S.M. & Rice,C.M. Expression and identification of hepatitis C virus polyprotein cleavage products. *J. Virol.* **67**, 1385-1395 (1993).

12. Hermanson,G.T. Bioconjugate techniques. Academic Press, San Diego (1996).

13. Holmgren,A. Thioredoxin catalyzes the reduction of insulin disulfides by dithiothreitol and dihydrolipoamide. *J. Biol. Chem.* **254**, 9627-9632 (1979).

14. Houghton,M. *et al.* Prospects for prophylactic and therapeutic hepatitis C virus vaccines. *Princess Takamatsu Symp.* **25**, 237-243 (1995).

15. Hu,G.J., Wang,R.Y., Han,D.S., Alter,H.J. & Shih,J.W. Characterization of the humoral and cellular immune responses against hepatitis C virus core induced by DNA-based immunization. *Vaccine* **17**, 3160-3170 (1999).

16. Ishak,K. *et al.* Histological grading and staging of chronic hepatitis. *J. Hepatol.* **22**, 696-699 (1995).

17. Johnson,D.A. *et al.* Synthesis and biological evaluation of a new class of vaccine adjuvants: aminoalkyl glucosaminide 4-phosphates (AGPs). *Bioorg. Med. Chem Lett* **9,** 2273-2278 (1999).

18. Knodell,R.G. *et al.* Formulation and application of a numerical scoring system for assessing histological activity in asymptomatic chronic active hepatitis. *Hepatology* **1**, 431-435 (1981).

19. Kumar,N., Kella,D. & Kinsella,J.E. A method for the controlled cleavage of disulfide bonds in proteins in the absence of denaturants. *J. Biochem. Biophys. Methods* **11**, 251-263 (1985).

20. Kumar,N., Kella,D. & Kinsella,J.E. Anomalous effects of denaturants on sulfitolysis of protein disulfide bonds. *Int. J. Peptide Prot. Res.* **28**, 586-592 (1986).

21. Lauer,G.M. & Walker,B.D. Hepatitis C virus infection. *N. Engl J. Med.* **345**, 41-52 (2001).

22. Lopez-Dias de Cerio AL *et al.* T(h)1 but not T(h)0 cell help is efficient to induce cytotoxic T lymphocytes by immunization with short synthetic peptides. *Int Immunol.* **11,** 2025-2034 (1999).

23. Persing,D. *et al.* Taking toll: lipid A mimetics as adjuvants and immunomodulators. *Trends Microbiol.* **10,** S32 (2002).

24. Pomroy,N.C. & Deber,C.M. Solubilization of hydrophobic peptides by reversible cysteine PEGylation. *Biochem. Biophys. Res. Commun.* **245,** 618-621 (1998).

25. Poynard,T., Bedossa,P. & Opolon,P. Natural history of liver fibrosis progression in patients with chronic hepatitis C. The OBSVIRC, METAVIR, CLINIVIR, and DOSVIRC groups. *Lancet* **349,** 825-832 (1997).

26. Rein,A. *et al.* Inactivation of murine leukemia virus by compounds that react with the zinc finger in the viral nucleocapsid protein. *J. Virol.* **70,** 4966-4972 (1996).

27. Riedl,P., Buschle,M., Reimann,J. & Schirmbeck,R. Binding immune-stimulating oligonucleotides to cationic peptides from viral core antigen enhances their potency as adjuvants. *Eur. J. Immunol.* **32**, 1709-1716 (2002).

28. Shiffman,M.L. Improvement in liver histopathology associated with interferon therapy in patients with chronic hepatitis C. *Viral Hepatitis Reviews* **5**,27-43 (1999).

29. Shimotohno,K. *et al.* Processing of the hepatitis C virus precursor protein. *J. Hepatol.* **22**, 87-92 (1995).

30. Shirai,M. *et al.* Use of intrinsic and extrinsic helper epitopes for in vivo induction of anti-hepatitis C virus cytotoxic T lymphocytes (CTL) with CTL epitope peptide vaccines. *J. Infect. Dis.* **173,** 24-31 (1996).

31. Singh,R. & Kats,L. Catalysis of reduction of disulfide by selenol. *Anal. Biochem.* **232***,* 86-91 (1995).

32. Song,M.K., Lee,S.W., Suh,Y.S., Lee,K.J. & Sung,Y.C. Enhancement of immunoglobulin G2a and cytotoxic T-lymphocyte responses by a booster immunization with recombinant hepatitis C virus E2 protein in E2 DNA-primed mice. *J. Virol.* **74***,* 2920-2925 (2000).

33. Thakur,M.L., DeFulvio,J., Richard,M.D. & Park,C.H. Technetium-99m labeled monoclonal antibodies: evaluation of reducing agents. *Int. J. Rad. Appl. Instrum. B* **18,** 227-233 (1991).

34. Uno-Furuta,S. *et al.* Induction of virus-specific cytotoxic T lymphocytes by in vivo electric administration of peptides. *Vaccine* **19,** 2190-2196 (2001).

35. Vingerhoeds,M.H. *et al.* Immunoliposomes as enzyme-carriers (immuno-enzymosomes) for antibody-directed enzyme prodrug therapy (ADEPT): optimization of prodrug activating capacity. *Pharm. Res.* **13,** 604-610 (1996).

36. Walewski,J.L., Keller,T.R., Stump,D.D. & Branch,A.D. Evidence for a new hepatitis C virus antigen encoded in an overlapping reading frame. *RNA.* **7**, 710-721 (2001).

37. Xu,Z. *et al.* Synthesis of a novel hepatitis C virus protein by ribosomal frameshift. *EMBO J.* **20,** 3840-3848 (2001).

## Claims

1. An isolated HCV NS5 protein or a part thereof wherein at least one cysteine thiol group is chemically or enzymatically modified in a reversible fashion.

2. The HCV NS5 protein or part thereof according to claim 1 comprising one or more amino acid derivatives, amino acid insertions, amino acid deletions or amino acid substitutions, or which is comprised in a fusion protein.

3. The HCV NS5 protein or part thereof according to claim 1 or 2 wherein said at least one cysteine thiol group is modified by sulphonation.

4. A composition comprising an HCV NS5 protein or part thereof according to any of claims 1 to 3 and at least one of a pharmaceutically acceptable carrier, adjuvant or vehicle.

5. The HCV composition according to claim 4 which is an HCV immunogenic composition, a prophylactic HCV vaccine composition or a therapeutic HCV vaccine composition.

6. The composition according to claim 5 further comprising a DNA vaccine vector.

7. A method for determining the presence of antibodies to HCV in a biological sample comprising the step of detecting said antibodies with an HCV NS5 protein or part thereof according to any of claims 1 to 3 wherein said detection is performed in the absence of a reducing agent.

**8.** A method for determining the presence of HCV NS5 antigens in a biological sample comprising the step of detecting said HCV NS5 antigens with an antibody to said HCV NS5 antigens in the presence of an HCV NS5 protein or part thereof according to any of claims 1 to 3 as competitor of binding of said HCV NS5 antigens to said antibody wherein said detection is performed in the absence of a reducing agent.

**9.** A diagnostic kit for determining the presence of antibodies to HCV in a biological sample, for determining the presence of HCV NS5 antigens in a biological sample, for determining the presence of a HCV virus in a biological sample or for diagnosing HCV infection in a mammal, said kit comprising an HCV NS5 protein or part thereof according to any of claims 1 to 3.

**10.** The composition according to claim 3 for inducing a humoral and/or cellular immune response in a mammal, for prophylactic protection of a mammal against subsequent HCV infection or for therapeutic treatment of a HCV-infected mammal.

**11.** The composition according to claim 4 for inducing, in combination with a DNA vaccine, a humoral and/or cellular immune response in a mammal, for prophylactic protection of a mammal against subsequent HCV infection or for therapeutic treatment of a HCV-infected mammal.

**12.** Use of the HCV NS5 protein or part thereof according to any of claims 1 to 3 for the manufacture of an HCV immunogenic composition or an HCV vaccine composition.

**13.** Use of the HCV NS5 protein or part thereof according to any of claims 1 to 3 in an immunoassay or for the manufacture of an immunoassay or an immunoassay kit.

**14.** A method for producing the HCV NS5 protein or part thereof according to any of claims 1 to 3, said method comprising the steps of:

(i) obtaining an HCV NS5 protein or part thereof by means of recombinant expression or chemical synthesis;
(ii) reversibly or irreversibly modifying at least one cysteine thiol group in the HCV NS5 protein or part thereof obtained in (i);
(iii) purifying the HCV NS5 protein or part thereof of (ii).

**Amended claims in accordance with Rule 86(2) EPC**

**1.** An isolated HCV NS5 protein or a part thereof wherein at least one cysteine thiol group is chemically or enzymatically modified in a reversible fashion.

**2.** The HCV NS5 protein or part thereof according to claim I comprising one or more amino acid derivatives, amino acid insertions, amino acid deletions or amino acid substitutions, or which is comprised in a fusion protein.

**3.** The HCV NS5 protein or part thereof according to claim 1 or 2 wherein said at least one cysteine thiol group is reversibly modified by sulphonation.

**4.** A composition comprising an HCV NS5 protein or part thereof according to any of claims I to 3 and at least one of a pharmaceutically acceptable carrier, adjuvant or vehicle.

**5.** The composition according to claim 4 which is an HCV immunogenic composition, a prophylactic HCV vaccine composition or a therapeutic HCV vaccine composition.

**6.** The composition according to claim 5 further comprising a DNA vaccine vector.

**7.** A method for determining the presence of antibodies to HCV in a biological sample comprising the step of detecting said antibodies with an HCV NS5 protein or part thereof according to any of claims 1 to 3 wherein said detection is performed in the absence of a reducing agent.

**8.** A method for determining the presence of HCV NS5 antigens in a biological sample comprising the step of detecting said HCV NS5 antigens with an antibody to said HCV NS5 antigens in the presence of an HCV NS5 protein or part thereof according to any of claims 1 to 3 as competitor of binding of said HCV NS5 antigens to said antibody wherein said detection is performed in the absence of a reducing agent.

**9.** A diagnostic kit for determining the presence of antibodies to HCV in a biological sample, for determining the presence of HCV NS5 antigens in a biological sample, for determining the presence of an HCV virus in a biological sample or for diagnosing HCV infection in a mammal, said kit comprising an HCV NS5 protein or part thereof according to any of claims 1 to 3.

**10.** The composition according to claim 4 for inducing a humoral and/or cellular immune response in a mammal, for prophylactic protection of a mammal against subsequent HCV infection or for therapeutic treatment of an HCV-infected mammal.

**11.** The composition according to claim 4 for inducing, in combination with a DNA vaccine, a humoral and/or cellular immune response in a mammal, for prophylactic protection of a mammal against subsequent HCV infection or for therapeutic treatment of an HCV-infected mammal.

**12.** Use of the HCV NS5 protein or part thereof according to any of claims 1 to 3 for the manufacture of an HCV immunogenic composition or an HCV vaccine composition.

**13.** Use of the HCV NS5 protein or part thereof according to any of claims 1 to 3 in an immunoassay or for the manufacture of an immunoassay or an immunoassay kit.

**14.** A method for producing the HCV NS5 protein or part thereof according to any of claims 1 to 3, said method comprising the steps of:

(i) obtaining an HCV NS5 protein or part thereof by means of recombinant expression or chemical synthesis;
(ii) reversibly modifying at least one cysteine thiol group in the HCV NS5 protein or part thereof obtained in (i);
(iii) purifying the HCV NS5 protein or part thereof of (ii).

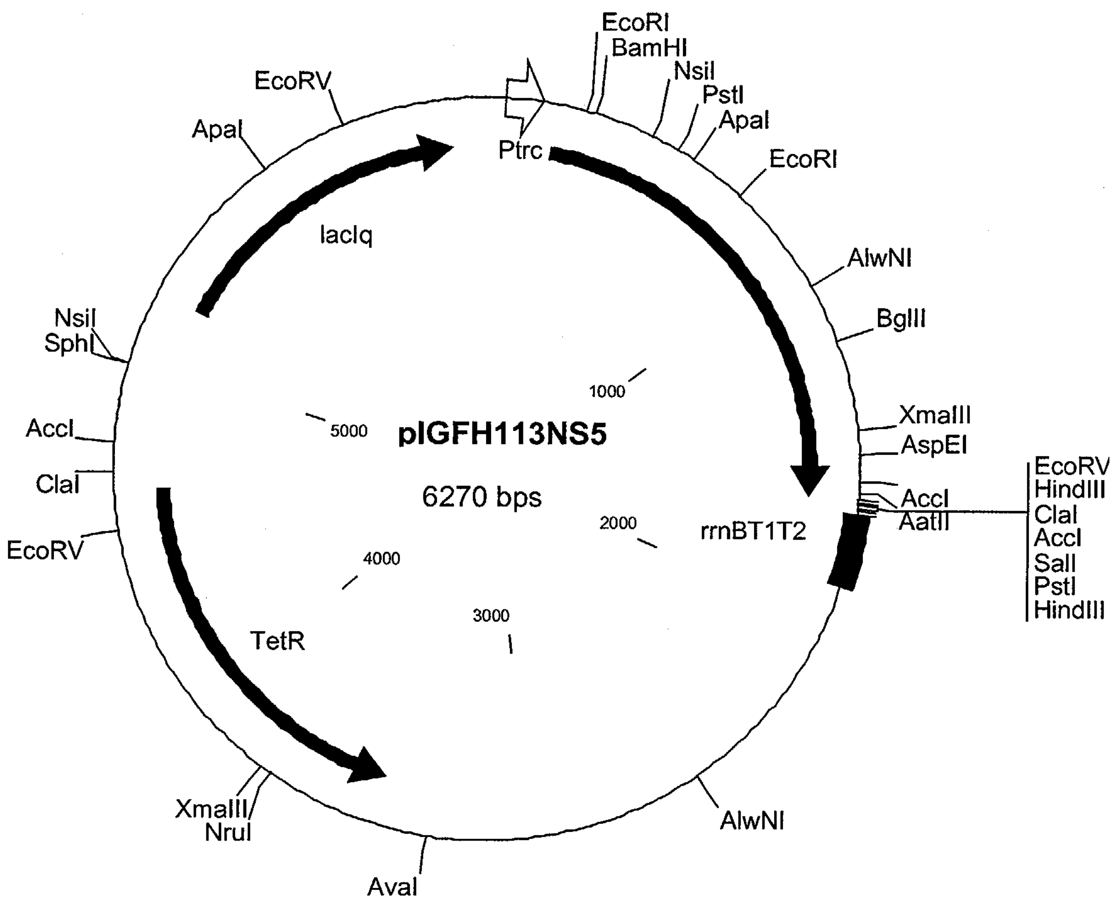
pIGFH113NS5
6270 bps
EcoRI
BamHI
NsiI
PstI
ApaI
EcoRI
AlwNI
BglII
XmaIII
AspEI
AccI
AatII
EcoRV
HindIII
ClaI
AccI
SalI
PstI
HindIII
rrnBT1T2
AlwNI
AvaI
XmaIII
NruI
TetR
EcoRV
ClaI
AccI
NsiI
SphI
ApaI
EcoRV
lacIq
Ptrc
1000
2000
3000
4000
5000

FIGURE 1

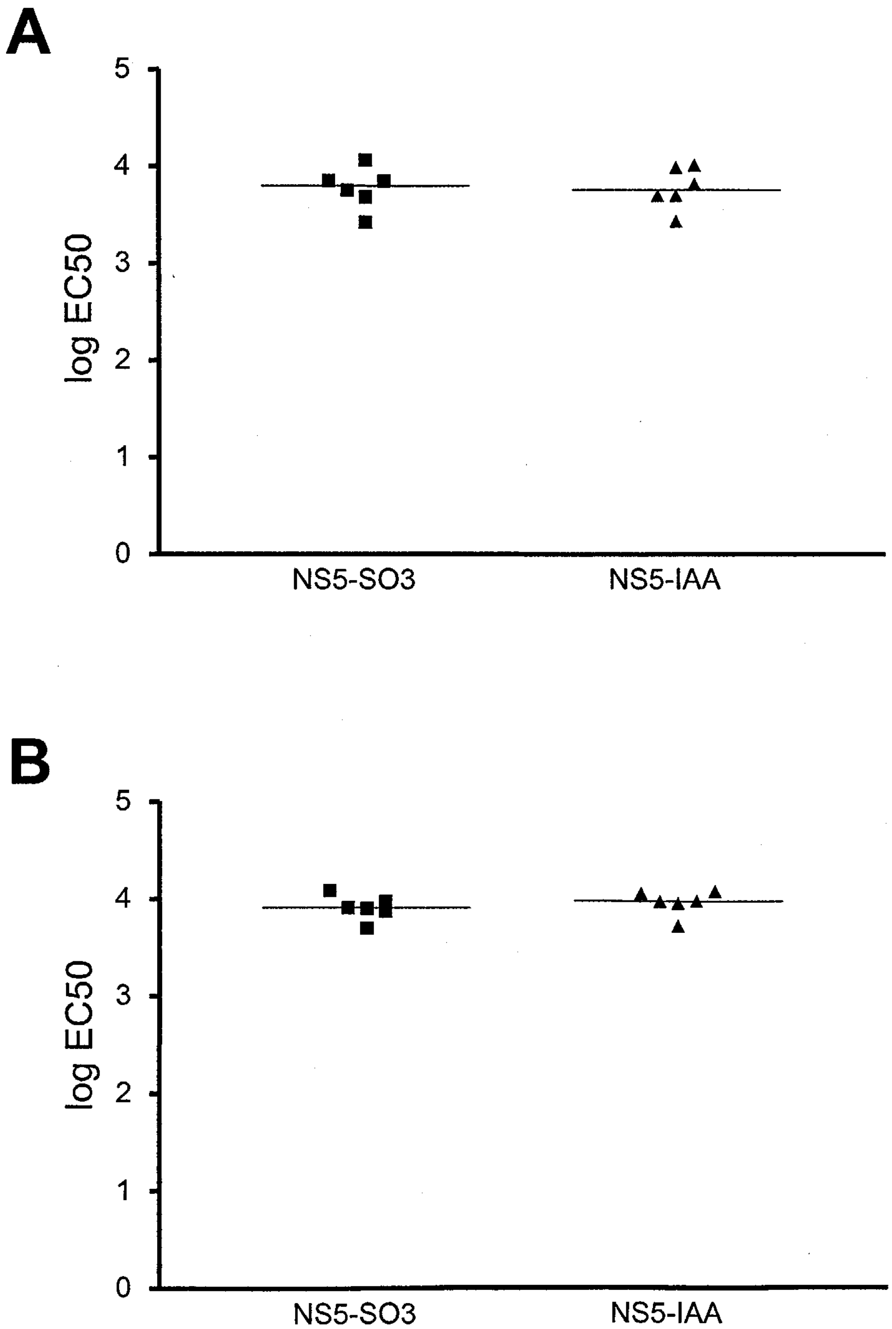
A
log EC50
5
4
3
2
1
0
NS5-SO3
NS5-IAA
B
log EC50
5
4
3
2
1
0
NS5-SO3
NS5-IAA

FIGURE 2

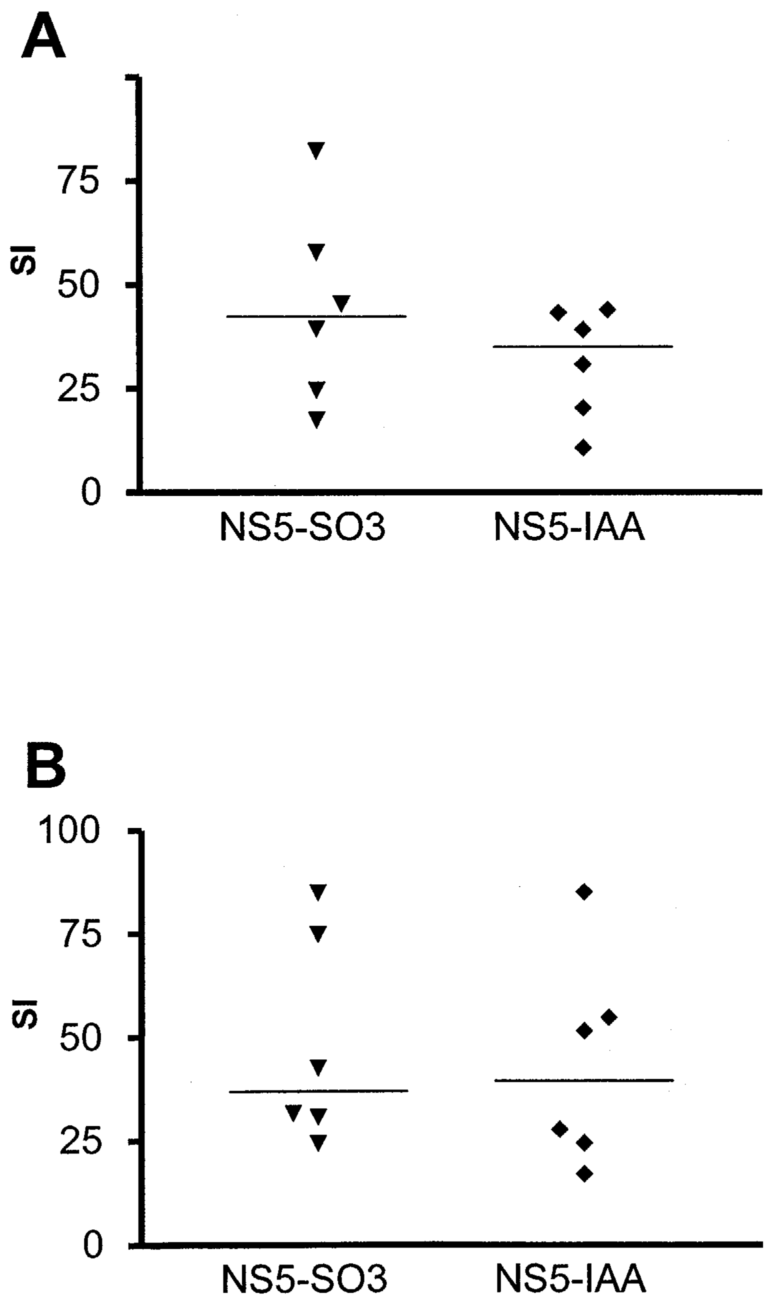


FIGURE 3

European Patent Office

# EUROPEAN SEARCH REPORT

Application Number
EP 03 44 7130

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 30815 A (BOSMAN ALFONS ;DEPLA ERIK (BE); INNOGENETICS NV (BE); MAERTENS GEE) 3 May 2001 (2001-05-03)<br>* abstract *<br>* page 1, line 6-10,36 *<br>* page 3, line 1-13 *<br>* page 4, line 24-26 *<br>* page 5, line 5-32 *<br>* page 9, line 8 - page 10, line 17 *<br>* page 13, line 10-13 *<br>* page 24, line 4-12 *<br>----- | 1-10 | C07K14/18<br>A61K39/29<br>A61P31/14<br>G01N33/53<br>G01N33/576 |
| | | | TECHNICAL FIELDS SEARCHED (Int.Cl.7) |
| | | | C07K |

~~The present search report has been drawn up for all claims~~

| Place of search | Date of completion of the search | Examiner |
|---|---|---|
| MUNICH | 5 September 2003 | Montrone, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

Application Number
EP 03 44 7130

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1, 2 (completely), 3-10 (all partially)

European Patent Office

# LACK OF UNITY OF INVENTION SHEET B

Application Number
EP 03 44 7130

The Search Division considers that the present European patent application does not comply with the requirements of unity of invention and relates to several inventions or groups of inventions, namely:

1. Claims: 1, 2 (completely), 3-10 (all partially)

   An isolated HCV NS5 protein or a part thereof wherein at least one cysteine thiol group is reversibly chemically or enzymatically modified.

2. Claims: 3-10 (all partially)

   An isolated HCV NS5 protein or a part thereof wherein at least one cysteine thiol group is irreversibly chemically or enzymatically modified.

## ANNEX TO THE EUROPEAN SEARCH REPORT ON EUROPEAN PATENT APPLICATION NO. EP 03 44 7130

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2003

| Patent document cited in search report | Publication date | Patent family member(s) | Publication date |
|---|---|---|---|
| WO 0130815 A | 03-05-2001 | AU 1144501 A | 08-05-2001 |
| | | BR 0015170 A | 25-06-2002 |
| | | CA 2387666 A1 | 03-05-2001 |
| | | CN 1384839 T | 11-12-2002 |
| | | CZ 20021819 A3 | 18-06-2003 |
| | | WO 0130815 A1 | 03-05-2001 |
| | | EP 1224214 A1 | 24-07-2002 |
| | | HU 0203195 A2 | 28-12-2002 |
| | | JP 2003513022 T | 08-04-2003 |
| | | NO 20021993 A | 27-06-2002 |
| | | NZ 518095 A | 26-09-2003 |

EPO FORM P0459

For more details about this annex : see Official Journal of the European Patent Office, No. 12/82

SEQUENCE LISTING

<110> Innogenetics N.V.

<120> Modified HCV NS5

<130> 153

<160> 6

<170> PatentIn version 3.1

<210> 1
<211> 300
<212> PRT
<213> hepatitis C virus

<220>
<221> MISC_FEATURE
<222> (5)..(5)
<223> Xaa may be Ile,Val or Thr

<220>
<221> MISC_FEATURE
<222> (8)..(8)
<223> Xaa may be Val or Ala

<400> 1

```
Met Ala Thr Cys Xaa Asn Gly Xaa Cys Trp Thr Val Tyr His Gly Arg
1               5                   10                  15

Ala Ala Val Cys Thr Arg Gly Val Ala Lys Ala Val Asp Phe Val Pro
            20                  25                  30

Val Glu Ser Met Glu Thr Thr Met Arg Ser Pro Val Phe Thr Asp Asn
        35                  40                  45
```

Ser Ser Pro Pro Ala Val Pro Gln Thr Phe Gln Val Ala His Leu His
    50                  55                  60

Ala Pro Thr Gly Ser Gly Lys Ser Thr Lys Val Pro Ala Ala Tyr Ala
65                  70                  75                  80

Ala Gln Gly Tyr Lys Val Leu Val Leu Asn Pro Ser Val Ala Ala Thr
                85                  90                  95

Leu Gly Phe Gly Ala Tyr Met Ser Lys Ala His Gly Val Asp Pro Asn
            100                 105                 110

Ile Arg Thr Gly Val Arg Thr Ile Thr Thr Gly Ala Pro Ile Thr Tyr
        115                 120                 125

Ser Thr Tyr Gly Lys Phe Leu Ala Asp Gly Gly Cys Ser Gly Gly Ala
    130                 135                 140

Tyr Asp Ile Ile Ile Cys Asp Glu Cys His Ser Ile Asp Ser Thr Ser
145                 150                 155                 160

Ile Leu Gly Ile Gly Thr Val Leu Asp Gln Ala Glu Thr Ala Gly Ala
                165                 170                 175

Arg Leu Val Val Leu Ala Thr Ala Thr Pro Pro Gly Ser Val Thr Val
            180                 185                 190

Pro His Pro Asn Ile Glu Glu Val Ala Leu Ser Ser Thr Gly Glu Ile
        195                 200                 205

Pro Phe Tyr Gly Lys Ala Ile Pro Ile Glu Val Ile Lys Gly Gly Arg
    210                 215                 220

His Leu Ile Phe Cys His Ser Lys Lys Lys Cys Asp Glu Leu Ala Ala
225                 230                 235                 240

Lys Leu Ser Gly Phe Gly Ile Asn Ala Val Ala Tyr Tyr Arg Gly Leu
                245                 250                 255

Asp Val Ser Val Ile Pro Thr Ser Gly Asp Val Val Val Val Ala Thr
            260                 265                 270

Asp Ala Leu Met Thr Gly Phe Thr Gly Asp Phe Asp Ser Val Ile Asp
        275                 280                 285

Cys Asn Thr Cys Val Thr Gln Thr Val Asp Phe Ser
    290                 295                 300

<210> 2

<211> 6270

<212> DNA

<213> Artificial sequence

<220>

<223> pIGFH113NS5

<400> 2
tatggctgtg caggtcgtaa atcactgcat aattcgtgtc gctcaaggcg cactcccgtt 60

ctggataatg ttttttgcgc cgacatcata acggttctgg caaatattct gaaatgagct 120

gttgacaatt aatcatccgg ctcgtataat gtgtggaatt gtgagcggat aacaatttca 180

cacaggaaac agaccatgtc aagtagtcaa aattcgagtg acaagcctgt agcccacgtc 240

gtagcaaacc accaagtgga ggagcaggga attcaccatc accatcacca cgtggatccc 300

tctggctcgt ggctcaggga cgtttgggac tggatatgca cggtgttgac tgacttcaag 360

acctggctcc agtccaagct cctgccacgg ttgccgggag tcccgttctt ctcatgccaa 420

cgtgggtaca agggagtctg gcggggagat ggcatcatgc ataccacctg cccatgtggg 480

gcacaaatca ctggacatgt caaaaacggc tccatgagga tcgttgggcc tagaacctgc 540

agcaacacgt ggcatggaac gttccccatc aacgcgtaca ccacgggccc ctgcacaccc 600

tccccggcgc caaattattc cagggcgctg tggcgggtgg ctgctgagga gtacgtggaa 660

attacgcggg tgggggactt ccactacgtg acgggcatga ccaccgacaa cgtaaagtgc 720

ccatgccagg ttccggcccc cgaattcttc acagaagtgg atggtgtacg gctgcacagg 780

tacgctccgg cgtgcaaacc tctcctgcgg gaggaggtca cattccaggt cgggctcaac 840

caatacctgg ttgggtcaca gctcccatgc gagcccgaac cggatgtagt ggtggttact 900

tccatgctca ccgacccctc ccacatcacg gcggagacgg ccaagcgtag gctggccagg 960

gggtctcccc cctccttggc cagctcatca gctagtcagc tgtctgcacc ttccttgaaa 1020

gcgacatgca ctacccatca cgactcccca gacgctgacc tcatcgaggc caacctcctg 1080

tggcggcagg agatgggcgg gaacatcacc cgcgtggagt cagagaataa ggtagtagtt 1140

ctggactctt ttgacccgct tcgagcggag gaagatgagg gggaagtatc cgttccggcg 1200

gagatcctgc ggagatctag gaagttcccc ccagcgatgc ccatatgggc gcgcccagat 1260

tacaaccctc cactactaga gtcctggaag gccccggact acgtccctcc ggtggtacac 1320

gggtgcccat tgccacctac gaaggccccc ccaataccac ctccacggag aaagcggaca 1380

gttgtcctaa cagaatccac cgtgtcctct gccttggcgg agcttgctac aaagaccttc 1440

ggcagctccg gttcgtcggc cgttgacagc ggtacggcga ccgcccctcc tgatcagccc 1500

tccgacgacg gcgacgcggg atcggacgtt gagtcgtact cctccatgcc ccccccttgag 1560

ggagagccgg gggaccccga tctcagcgac gggtcttggt ctaccgtgag cgaggaggct 1620

agtgaggacg tcgtctgctg cgcatgatat ccgatcaagc ttatcgatac cgtcgacctg 1680

cagccaagct tggctgtttt ggcggatgag agaagatttt cagcctgata cagattaaat 1740

cagaacgcag aagcggtctg ataaaacaga atttgcctgg cggcagtagc gcggtggtcc 1800
cacctgaccc catgccgaac tcagaagtga aacgccgtag cgccgatggt agtgtggggt 1860
ctccccatgc gagagtaggg aactgccagg catcaaataa aacgaaaggc tcagtcgaaa 1920
gactgggcct ttcgttttat ctgttgtttg tcggtgaacg ctctcctgag taggacaaat 1980
ccgccgggag cggatttgaa cgttgcgaag caacggcccg gagggtggcg ggcaggacgc 2040
ccgccataaa ctgccaggca tcaaattaag cagaaggcca tcctgacgga tggccttttt 2100
gcgtttctac aaactctttt gtttattttt ctaaatacat tcaaatatgt atccgctcat 2160
gagacaataa ccctgataaa tgcttcaata ataaaaggat ctaggtgaag atcctttttg 2220
ataatctcat gaccaaaatc ccttaacgtg agttttcgtt ccactgagcg tcagaccccg 2280
tagaaaagat caaaggatct tcttgagatc cttttttct gcgcgtaatc tgctgcttgc 2340
aaacaaaaaa accaccgcta ccagcggtgg tttgtttgcc ggatcaagag ctaccaactc 2400
tttttccgaa ggtaactggc ttcagcagag cgcagatacc aaatactgtc cttctagtgt 2460
agccgtagtt aggccaccac ttcaagaact ctgtagcacc gcctacatac ctcgctctgc 2520
taatcctgtt accagtggct gctgccagtg gcgataagtc gtgtcttacc gggttggact 2580
caagacgata gttaccggat aaggcgcagc ggtcgggctg aacggggggt tcgtgcacac 2640
agcccagctt ggagcgaacg acctacaccg aactgagata cctacagcgt gagcattgag 2700
aaagcgccac gcttcccgaa gggagaaagg cggacaggta tccggtaagc ggcagggtcg 2760
gaacaggaga gcgcacgagg gagcttccag ggggaaacgc ctggtatctt tatagtcctg 2820
tcgggtttcg ccacctctga cttgagcgtc gatttttgtg atgctcgtca ggggggcgga 2880
gcctatggaa aaacgccagc aacgcggcct ttttacggtt cctggccttt tgctggcctt 2940
ttgctcacat gttctttcct gcgttatccc ctgattctgt ggataaccgt attaccgcct 3000
ttgagtgagc tgataccgct cgccgcagcc gaacgaccga gcgcagcgag tcagtgagcg 3060
aggaagcgga agagcgctga cttccgcgtt tccagacttt acgaaacacg gaaaccgaag 3120
accattcatg ttgttgctca ggtcgcagac gttttgcagc agcagtcgct tcacgttcgc 3180
tcgcgtatcg gtgattcatt ctgctaacca gtaaggcaac cccgccagcc tagccgggtc 3240
ctcaacgaca ggagcacgat catgcgcacc cgtggccagg acccaacgct gcccgagatg 3300
cgccgcgtgc ggctgctgga gatggcggac gcgatggata tgttctgcca agggttggtt 3360
tgcgcattca cagttctccg caagaattga ttggctccaa ttcttggagt ggtgaatccg 3420
ttagcgaggt gccgccggct tccattcagg tcgaggtggc ccggctccat gcaccgcgac 3480
gcaacgcggg gaggcagaca aggtataggg cggcgcctac aatccatgcc aacccgttcc 3540
atgtgctcgc cgaggcggca taaatcgccg tgacgatcag cggtccagtg atcgaagtta 3600
ggctggtaag agccgcgagc gatccttgaa gctgtccctg atggtcgtca tctacctgcc 3660
tggacagcat ggcctgcaac gcgggcatcc cgatgccgcc ggaagcgaga agaatcataa 3720
tggggaaggc catccagcct cgcgtcgcga acgccagcaa gacgtagccc agcgcgtcgg 3780

```
ccgccatgcc ggcgataatg gcctgcttct cgccgaaacg tttggtggcg ggaccagtga 3840
cgaaggcttg agcgagggcg tgcaagattc cgaataccgc aagcgacagg ccgatcatcg 3900
tcgcgctcca gcgaaagcgg tcctcgccga aaatgaccca gagcgctgcc ggcacctgtc 3960
ctacgagttg catgataaag aagacagtca taagtgcggc gacgatagtc atgccccgcg 4020
cccaccggaa ggagctgact gggttgaagg ctctcaaggg catcggtcgg cgctctccct 4080
tatgcgactc ctgcattagg aagcagccca gtagtaggtt gaggccgttg agcaccgccg 4140
ccgcaaggaa tggtgcatgt aaggagatgg cgcccaacag tcccccggcc acggggcctg 4200
ccaccatacc cacgccgaaa caagcgctca tgagcccgaa gtggcgagcc cgatcttccc 4260
catcggtgat gtcggcgata taggcgccag caaccgcacc tgtggcgccg gtgatgccgg 4320
ccacgatgcg tccggcgtag agaatccaca ggacgggtgt ggtcgccatg atcgcgtagt 4380
cgatagtggc tccaagtagc gaagcgagca ggactgggcg gcggccaaag cggtcggaca 4440
gtgctccgag aacgggtgcg catagaaatt gcatcaacgc atatagcgct agcagcacgc 4500
catagtgact ggcgatgctg tcggaatgga cgatatcccg caagaggccc ggcagtaccg 4560
gcataaccaa gcctatgcct acagcatcca gggtgacggt gccgaggatg acgatgagcg 4620
cattgttaga tttcatacac ggtgcctgac tgcgttagca atttaactgt gataaactac 4680
cgcattaaag ctaatcgatg ataagctgtc aaacatgaga atttatggtg cactctcagt 4740
acaatctgct ctgatgccgc atagttaagc cagtatacac tccgctatcg ctacgtgact 4800
gggtcatggc tgcgccccga cacccgccaa cacccgctga cgcgccctga cgggcttgtc 4860
tgctcccggc atccgcttac agacaagctg tgaccgtctc cgggagctgc atgtgtcaga 4920
ggttttcacc gtcatcaccg aaacgcgcga ggcagcagat caattcgcgc gcgaaggcga 4980
agcggcatgc atttacgttg acaccatcga atggtgcaaa acctttcgcg gtatggcatg 5040
atagcgcccg gaagagagtc aattcagggt ggtgaatgtg aaaccagtaa cgttatacga 5100
tgtcgcagag tatgccggtg tctcttatca gaccgtttcc cgcgtggtga accaggccag 5160
ccacgtttct gcgaaaacgc gggaaaaagt ggaagcggcg atggcggagc tgaattacat 5220
tcccaaccgc gtggcacaac aactggcggg caaacagtcg ttgctgattg gcgttgccac 5280
ctccagtctg gccctgcacg cgccgtcgca aattgtcgcg gcgattaaat ctcgcgccga 5340
tcaactgggt gccagcgtgg tggtgtcgat ggtagaacga agcggcgtcg aagcctgtaa 5400
agcggcggtg cacaatcttc tcgcgcaacg cgtcagtggg ctgatcatta actatccgct 5460
ggatgaccag gatgccattg ctgtggaagc tgcctgcact aatgttccgg cgttatttct 5520
tgatgtctct gaccagacac ccatcaacag tattattttc tcccatgaag acggtacgcg 5580
actgggcgtg gagcatctgg tcgcattggg tcaccagcaa atcgcgctgt tagcgggccc 5640
attaagttct gtctcggcgc gtctgcgtct ggctggctgg cataaatatc tcactcgcaa 5700
tcaaattcag ccgatagcgg aacgggaagg cgactggagt gccatgtccg gttttcaaca 5760
aaccatgcaa atgctgaatg agggcatcgt tcccactgcg atgctggttg ccaacgatca 5820
```

gatggcgctg ggcgcaatgc gcgccattac cgagtccggg ctgcgcgttg gtgcggatat 5880
ctcggtagtg ggatacgacg ataccgaaga cagctcatgt tatatcccgc cgtcaaccac 5940
catcaaacag gattttcgcc tgctggggca aaccagcgtg gaccgcttgc tgcaactctc 6000
tcagggccag gcggtgaagg gcaatcagct gttgcccgtc tcactggtga aaagaaaaac 6060
caccctggcg cccaatacgc aaaccgcctc tccccgcgcg ttggccgatt cattaatgca 6120
gctggcacga caggtttccc gactggaaag cgggcagtga gcgcaacgca attaatgtga 6180
gttagcgcga attgatctgg tttgacagct tatcatcgac tgcacggtgc accaatgctt 6240
ctggcgtcag gcagccatcg gaagctgtgg 6270

<210> 3
<211> 483
<212> PRT
<213> hepatitis C virus

<400> 3

Met Ser Ser Ser Gln Asn Ser Ser Asp Lys Pro Val Ala His Val Val
1 5 10 15

Ala Asn His Gln Val Glu Glu Gln Gly Ile His His His His His His
20 25 30

Val Asp Pro Ser Gly Ser Trp Leu Arg Asp Val Trp Asp Trp Ile Cys
35 40 45

Thr Val Leu Thr Asp Phe Lys Thr Trp Leu Gln Ser Lys Leu Leu Pro
50 55 60

Arg Leu Pro Gly Val Pro Phe Phe Ser Cys Gln Arg Gly Tyr Lys Gly
65 70 75 80

Val Trp Arg Gly Asp Gly Ile Met His Thr Thr Cys Pro Cys Gly Ala
85 90 95

Gln Ile Thr Gly His Val Lys Asn Gly Ser Met Arg Ile Val Gly Pro
100 105 110

Arg Thr Cys Ser Asn Thr Trp His Gly Thr Phe Pro Ile Asn Ala Tyr
115 120 125

Thr Thr Gly Pro Cys Thr Pro Ser Pro Ala Pro Asn Tyr Ser Arg Ala
130 135 140

Leu Trp Arg Val Ala Ala Glu Glu Tyr Val Glu Ile Thr Arg Val Gly
145 150 155 160

```
Asp Phe His Tyr Val Thr Gly Met Thr Thr Asp Asn Val Lys Cys Pro
                165                 170                 175

Cys Gln Val Pro Ala Pro Glu Phe Phe Thr Glu Val Asp Gly Val Arg
            180                 185                 190

Leu His Arg Tyr Ala Pro Ala Cys Lys Pro Leu Leu Arg Glu Glu Val
        195                 200                 205

Thr Phe Gln Val Gly Leu Asn Gln Tyr Leu Val Gly Ser Gln Leu Pro
    210                 215                 220

Cys Glu Pro Glu Pro Asp Val Val Val Val Thr Ser Met Leu Thr Asp
225                 230                 235                 240

Pro Ser His Ile Thr Ala Glu Thr Ala Lys Arg Arg Leu Ala Arg Gly
                245                 250                 255

Ser Pro Pro Ser Leu Ala Ser Ser Ser Ala Ser Gln Leu Ser Ala Pro
            260                 265                 270

Ser Leu Lys Ala Thr Cys Thr Thr His His Asp Ser Pro Asp Ala Asp
        275                 280                 285

Leu Ile Glu Ala Asn Leu Leu Trp Arg Gln Glu Met Gly Gly Asn Ile
    290                 295                 300

Thr Arg Val Glu Ser Glu Asn Lys Val Val Val Leu Asp Ser Phe Asp
305                 310                 315                 320

Pro Leu Arg Ala Glu Glu Asp Glu Gly Glu Val Ser Val Pro Ala Glu
                325                 330                 335

Ile Leu Arg Arg Ser Arg Lys Phe Pro Pro Ala Met Pro Ile Trp Ala
            340                 345                 350

Arg Pro Asp Tyr Asn Pro Pro Leu Leu Glu Ser Trp Lys Ala Pro Asp
        355                 360                 365

Tyr Val Pro Pro Val Val His Gly Cys Pro Leu Pro Pro Thr Lys Ala
    370                 375                 380

Pro Pro Ile Pro Pro Pro Arg Arg Lys Arg Thr Val Val Leu Thr Glu
385                 390                 395                 400

Ser Thr Val Ser Ser Ala Leu Ala Glu Leu Ala Thr Lys Thr Phe Gly
                405                 410                 415

Ser Ser Gly Ser Ser Ala Val Asp Ser Gly Thr Ala Thr Ala Pro Pro
            420                 425                 430
```

Asp Gln Pro Ser Asp Asp Gly Asp Ala Gly Ser Asp Val Glu Ser Tyr
435 440 445

Ser Ser Met Pro Pro Leu Glu Gly Glu Pro Gly Asp Pro Asp Leu Ser
450 455 460

Asp Gly Ser Trp Ser Thr Val Ser Glu Glu Ala Ser Glu Asp Val Val
465 470 475 480

Cys Cys Ala

<210> 4

<211> 448

<212> PRT

<213> hepatitis C virus

<400> 4

Ser Gly Ser Trp Leu Arg Asp Val Trp Asp Trp Ile Cys Thr Val Leu
1 5 10 15

Thr Asp Phe Lys Thr Trp Leu Gln Ser Lys Leu Leu Pro Arg Leu Pro
20 25 30

Gly Val Pro Phe Phe Ser Cys Gln Arg Gly Tyr Lys Gly Val Trp Arg
35 40 45

Gly Asp Gly Ile Met His Thr Thr Cys Pro Cys Gly Ala Gln Ile Thr
50 55 60

Gly His Val Lys Asn Gly Ser Met Arg Ile Val Gly Pro Arg Thr Cys
65 70 75 80

Ser Asn Thr Trp His Gly Thr Phe Pro Ile Asn Ala Tyr Thr Thr Gly
85 90 95

Pro Cys Thr Pro Ser Pro Ala Pro Asn Tyr Ser Arg Ala Leu Trp Arg
100 105 110

Val Ala Ala Glu Glu Tyr Val Glu Ile Thr Arg Val Gly Asp Phe His
115 120 125

Tyr Val Thr Gly Met Thr Thr Asp Asn Val Lys Cys Pro Cys Gln Val
130 135 140

Pro Ala Pro Glu Phe Phe Thr Glu Val Asp Gly Val Arg Leu His Arg
145 150 155 160

```
Tyr Ala Pro Ala Cys Lys Pro Leu Leu Arg Glu Glu Val Thr Phe Gln
                165                 170                 175

Val Gly Leu Asn Gln Tyr Leu Val Gly Ser Gln Leu Pro Cys Glu Pro
            180                 185                 190

Glu Pro Asp Val Val Val Val Thr Ser Met Leu Thr Asp Pro Ser His
        195                 200                 205

Ile Thr Ala Glu Thr Ala Lys Arg Arg Leu Ala Arg Gly Ser Pro Pro
    210                 215                 220

Ser Leu Ala Ser Ser Ser Ala Ser Gln Leu Ser Ala Pro Ser Leu Lys
225                 230                 235                 240

Ala Thr Cys Thr Thr His His Asp Ser Pro Asp Ala Asp Leu Ile Glu
                245                 250                 255

Ala Asn Leu Leu Trp Arg Gln Glu Met Gly Gly Asn Ile Thr Arg Val
            260                 265                 270

Glu Ser Glu Asn Lys Val Val Val Leu Asp Ser Phe Asp Pro Leu Arg
        275                 280                 285

Ala Glu Glu Asp Glu Gly Glu Val Ser Val Pro Ala Glu Ile Leu Arg
    290                 295                 300

Arg Ser Arg Lys Phe Pro Pro Ala Met Pro Ile Trp Ala Arg Pro Asp
305                 310                 315                 320

Tyr Asn Pro Pro Leu Leu Glu Ser Trp Lys Ala Pro Asp Tyr Val Pro
                325                 330                 335

Pro Val Val His Gly Cys Pro Leu Pro Pro Thr Lys Ala Pro Pro Ile
            340                 345                 350

Pro Pro Pro Arg Arg Lys Arg Thr Val Val Leu Thr Glu Ser Thr Val
        355                 360                 365

Ser Ser Ala Leu Ala Glu Leu Ala Thr Lys Thr Phe Gly Ser Ser Gly
    370                 375                 380

Ser Ser Ala Val Asp Ser Gly Thr Ala Thr Ala Pro Pro Asp Gln Pro
385                 390                 395                 400

Ser Asp Asp Gly Asp Ala Gly Ser Asp Val Glu Ser Tyr Ser Ser Met
                405                 410                 415

Pro Pro Leu Glu Gly Glu Pro Gly Asp Pro Asp Leu Ser Asp Gly Ser
            420                 425                 430
```

```
Trp Ser Thr Val Ser Glu Glu Ala Ser Glu Asp Val Val Cys Cys Ala
        435                 440                 445

<210>  5

<211>  29

<212>  DNA

<213>  Artificial sequence


<220>

<223>  HCPr5065

<400>  5
gctcttcatc tggctcgtgg ctcagggac                                     29

<210>  6

<211>  28

<212>  DNA

<213>  Artificial sequence


<220>

<223>  HCPr5066

<400>  6
tctagaatca tgcgcagcag acgacgtc                                      28
```